# EUROPEAN PATENT APPLICATION

(11) **EP 2 031 405 A1**
(43) Date of publication of application: **04.03.2009**
(21) Application number: 08159294.1
(22) Date of filing: 27.06.2008
(51) Int. Cl.: G01N 35/00, G01N 35/10

(54) **Apparatus and method for actuating a syringe**

(30) Priority: 29.06.2007 US 771824
(71) Applicant: Symyx Technologies, Inc., Sunnyvale CA 94085 (US)
(72) Inventor: Higashihara, Kenneth, Sunnyvale, CA 94085 (US); Yoder, Jeffrey, Sunnyvale, CA 94085 (US); Nguyen, Tuyen, Victoria British Columbia V8T 3E9 (CA); Whiting, Scott, Sunnyvale, CA 94085 (US); Myslovaty, Michael, Sunnyvale, CA 94085 (US)
(74) Representative: Eisenführ, Speiser & Partner

(57) **Abstract**

A robotic workstation includes apparatus for actuating one or more syringes. Each syringe has a cylinder and a plunger movable in the cylinder. A programmable robot has at least one syringe actuating device. The actuating device has a holder for holding and releasing a syringe cylinder and an actuator movable relative to the holder. A coupling couples the actuator to the plunger of the syringe such that, when the cylinder is held by the holder, movement of the actuator relative to the holder causes the plunger to move in the cylinder. A method of dispensing a material into a container includes robotically moving at least one syringe to a position for dispensing into a container. The plunger is robotically moved in the cylinder to dispense material from the syringe into the container. The workstation suitably includes a mixing apparatus for mixing materials in an array of containers at the workstation.

## Description

### FIELD OF THE INVENTION

This invention relates to apparatus and methods for handling a syringe, and more particularly to such apparatus and methods which involves the use of a programmable robot and related accessories to automate a workflow involving dispensing material from a syringe and/or aspirating material into a syringe.

### BACKGROUND

Automation is well established in the field of materials discovery and research. Over the past several years, there have been efforts to apply automation and high throughput techniques into various development labs in which automated systems have been set up to serve dedicated workflows. For example, there are a number of automated reactor systems that have been used for synthesis screening and process optimization. See, for example, J. Am. Chem. Soc. 2003, 125, 4306-4317; "An Automated Approach to Process Optimization, Parameter Setting, and Robustness Testing" Organic Process R&D 2001, 5, 331-334; J. Am. Chem. Soc. 2002, 124, 15280 15285; "Automated Workstations for Parallel Synthesis" Organic Process R&D 2002, 6, 833-840; "Parallel solid-phase synthesis, screening, and encoding strategies for olefin-polymerization catalysts." Tetrahedron 1999, 55(39), 11699-11710; "An integrated high-throughput workflow for pre-formulations: Polymorphs and salt selection studies" Pharmachem, 2003, 1(7/8); and "Application of high throughput technologies to drug substance and drug product development" Computers and Chem. Eng. 2004, 28, 943-953.

The above efforts include procedures intended to automate the preparation of various formulations. See, e.g., published US patent application Ser. No. 10/448,788, published April 15, 2004 (Publication No. 2004/0071888); and published US patent application Ser. No. 9/682,829, published April 24, 2003 (Publication No. 2003/0677390).

While these examples highlight that automation has been successfully applied to dedicated workflows, there is a need for even more flexible and efficient automation systems.

### SUMMARY

In one aspect, this invention is directed to apparatus for actuating one or more syringes, each syringe comprising a cylinder having upper and lower ends and a plunger movable in the cylinder. The apparatus includes a programmable robot. At least one syringe actuating device is mounted on the robot for actuating a syringe to transfer material to or from a container. The syringe actuating device has a holder for holding and releasing the cylinder of a syringe, an actuator movable relative to the holder, and a coupling for coupling the actuator to the plunger of the syringe. When the cylinder of the syringe is held by the holder, movement of the actuator in a first direction causes the plunger to move up in the cylinder and movement of the actuator in a second direction causes the plunger to move down in the cylinder.

Another aspect of this invention is apparatus adapted for use with a programmable robot to actuate one or more syringes comprising a cylinder having upper and lower ends and a plunger movable up and down in the cylinder. The apparatus has at least one syringe actuating device adapted to be mounted on a robot. The syringe actuating device has a holder for holding the cylinder of the syringe. The holder has at least one holding member movable between a holding position for holding the cylinder and a release position for releasing the cylinder. The syringe actuating device includes an actuator for moving the plunger of the syringe in the cylinder while the cylinder is held by the holder and a coupling for coupling the actuator to the plunger of the syringe such that upward movement of the actuator causes the plunger to move up in the cylinder and downward movement of the actuator causes the plunger to move down in the cylinder.

Yet another aspect of the invention is a method of robotically dispensing material from a syringe. The method includes robotically moving at least one syringe to a position for dispensing into a container. A plunger of the at least one syringe is robotically moved to dispense material from the syringe into the container. A property of the material dispensed into the container is sensed robotically.

Still another aspect of the invention is a method of robotically dispensing from two or more syringes. The method includes robotically moving two or more syringes to a location for dispensing or aspirating materials. Plungers of the two or more syringes are robotically moved in parallel for dispensing or aspirating material.

Yet another aspect of the present invention is a method of robotically actuating disposable syringes. Each syringe has a cylinder and a plunger movable in the cylinder. The method includes placing a plurality of disposable syringes in a rack. A selected syringe is robotically removed from the rack and transported to a material transfer station. The plunger of the syringe is robotically moved in the cylinder to transfer material to or from the syringe. The syringe is disposed of robotically.

Another aspect of the invention is a method of mixing materials. The method includes mixing material in a container having a bottom wall and an open top by rotating at least one mixing blade positioned inside the container above the bottom wall. The at least one mixing blade is caused to stop at a predetermined rotary position to provide a predetermined unobstructed path extending from the open top of the container to the bottom wall of the container. An aspirator is robotically moved down along said predetermined unobstructed path to an aspirating position adjacent the bottom wall of the container, taking into account the predetermined rotary position of the at least one mixing blade so that there is no contact between the blade and the aspirator as the aspirator moves to said aspirating position. The material is aspirated from the container.

Another aspect of the invention is a robotic work station for mixing materials. The workstation includes a robot. An aspirator is carried by the robot. A container at a mixing station for holding materials to be mixed has a bottom wall and an open top. A mixer for mixing the contents of the container has at least one mixing blade positioned inside the container above the bottom wall of the container. The work station includes a drive system for rotating the at least one mixing blade and a controller for controlling the drive system to stop the at least one mixing blade in the container at a predetermined rotary position to provide an unobstructed path extending from the open top of the container to the bottom wall of the container. The controlling controls the robot to move the aspirator down along said unobstructed path to an aspirating position adjacent the bottom wall of the container, taking into account the predetermined rotary position of the at least one mixing blade so that there is no contact between the blade and the aspirator as the aspirator moves to said aspirating position.

The details of embodiments of the invention are set forth in the accompanying claims, drawings and description, below. Other features, objects, and benefits of the invention will be apparent from the description and drawings.

### DESCRIPTION OF DRAWINGS

FIG. 1 is a perspective of an automated workstation incorporating apparatus of the present invention;

FIG. 2 is an enlarged portion of Fig. 1 showing, among other things one embodiment of a syringe rack of the workstation;

FIG. 3 is a cross section of a portion of the syringe rack taken in a plane including the line 3--3 on Fig. 2;

FIG. 4 is a perspective of one embodiment of a mixing module of the work station;

FIG. 5 is a top view of the mixing module shown in Fig. 4;

FIG. 6 is a cross section of the mixing module shown in Figs. 4 & 5 taken in a plane including the line 6--6 on Fig. 5;

FIG. 7 is an enlarged cross section of the mixing module shown in Figs. 4-6 taken in the same plane as the cross section shown in Fig. 6;

FIG. 8 is a perspective of the mixing module shown in Figs. 4-7 with parts removed to show drive shafts of a mixing system;

FIG. 9 is a perspective similar to Fig. 8 of the mixing module shown in Figs. 4-8 with part of a bearing plate broken away to show part of a belt and pulley system that drives the mixing shafts;

FIG.10 is a perspective similar to Fig. 9 of the mixing module shown in Figs. 4-9 with the entire bearing plate removed to show more of the belt and pulley system;

FIG. 11 is a perspective similar to Fig. 10 of the mixing module shown in Figs. 4-10 with part of a deck panel broken away to show a motor that drives the belt and pulley system;

FIG. 12 is a perspective of one embodiment of a weigh station of the workstation;

FIG. 13 is a side elevation of the weigh station shown in Fig. 12;

FIG. 14 is a cross section of a portion of the weigh station shown in Figs. 12-13 taken in a plane including line 14--14 on Fig. 12;

FIG. 15 is a perspective of one embodiment of a container gripping mechanism;

FIG. 16 is a perspective of one embodiment of a syringe actuating apparatus mounted on a robot arm;

FIG. 17 is a perspective of the syringe actuating apparatus shown in Fig. 16 in isolation from the robot arm;

FIG. 18 is a front elevation of the syringe actuating apparatus shown in Figs. 16 & 17;

FIG. 19 is a side elevation of the syringe actuating apparatus shown in Figs. 16-18;

FIG. 20 is a perspective of the syringe actuating apparatus shown in Figs. 16-19 with a syringe actuating device removed;

FIG. 21 is a perspective of one embodiment of a syringe actuating device of the syringe actuating apparatus shown in Figs. 16-20;

FIG. 22 is a perspective of the syringe actuating device shown in Fig. 21 in cross section taken in a plane including line 21--21 on Fig. 21;

FIGS. 23-27 are enlarged cross sections of portions of the syringe actuating device illustrating the syringe actuating device picking up, using and disposing of a syringe;

FIG. 28 is a perspective of one embodiment of holding members for holding a cylinder of a syringe, gripping members for gripping a plunger of the syringe, and a linkage system for coordinating release of the syringe by the holding and gripping members;

FIG. 29 is a perspective of another embodiment of a mixing station that can be used in combination with the workstation shown in Fig. 1;

FIGS. 30A is a perspective of a one embodiment of a mixing module of the mixing station shown in Fig. 29 and a robotic cap lifting mechanism for lifting a cap off of the containers;

FIGS. 30B-C are cross sections of the a portion of the mixing module illustrating a sequence in which the cap lifting mechanism lifts a cap off of a container;

FIG. 30D is a perspective of the mixing module shown in Fig. 30B showing another embodiment of a robotic container gripping mechanism lifting one of the containers from the mixing module;

FIG. 31 is a perspective of the mixing module shown in Fig. 30 with parts removed to show a heating system for heating the contents of containers in the mixing module;

FIG. 32 is a perspective of a portion of the mixing module shown in Figs. 30-31 in cross section taken in an plane include line 32--32 on Fig. 29;

FIG. 33 is a side elevation of the portion of the mixing module shown in Fig. 32 in cross section;

FIG. 34 is a perspective of the mixing station shown in Fig 29 with two of the mixing modules removed to show the drive shafts of a mixing system;

FIG. 35 is a perspective of the mixing station shown in Figs. 29 and 34 with additional parts removed to show drive systems of the mixing systems for the mixing modules; and

FIG. 36 is a perspective of the mixing station shown in Figs. 20 and 34-35 with the mixing shafts removed to better illustrate belts and pulleys of the drive systems for the mixing modules.

Corresponding parts are designated by corresponding reference numbers throughout the drawings.

### DETAILED DESCRIPTION

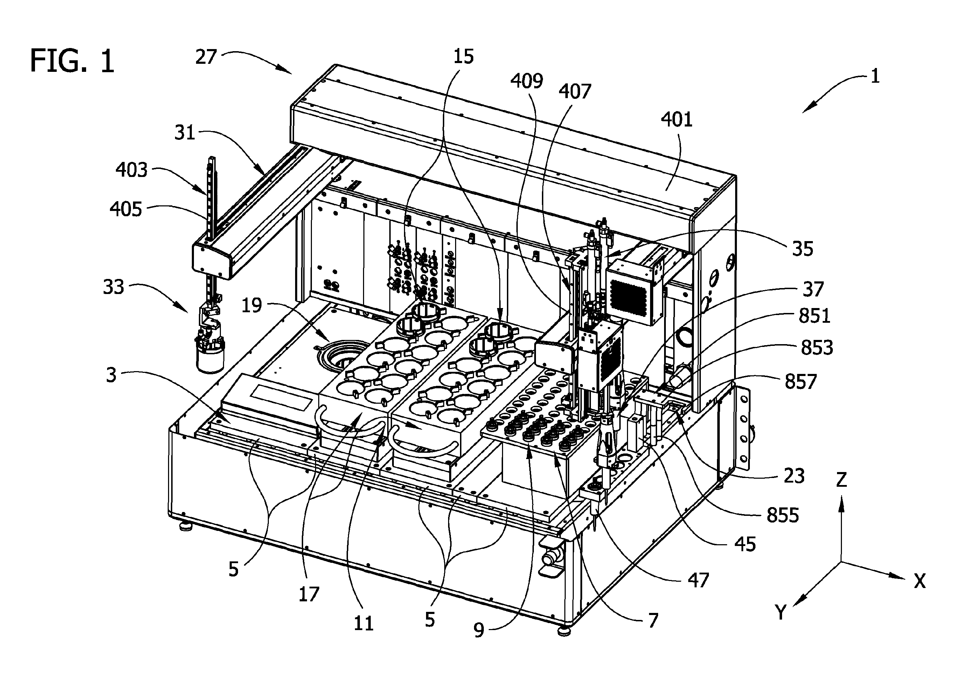

Fig. 1 illustrates an automated workstation, generally designated 1, for preparing various formulations of materials, particularly viscous materials. In general, the workstation 1 includes a deck 3 comprising a series of deck panels 5 mounted side-by-side, a rack 7 on the deck for holding an array of syringes 9 for aspirating and dispensing materials, a mixing station 11 at which formulations are prepared in a plurality of open-top containers 15 received in mixing modules 17, a weigh station 19 comprising weighing apparatus 21 for weighing the containers and their contents, and a syringe disposal station 23.

The syringes 9 and containers 15 are moved from one location to another by a programmable robot 27 having a first arm 31 which carries a gripping mechanism 33 for gripping a container 15 and moving it between the mixing and weighing stations 11, 19, and a second arm 35 which carries apparatus 37 for moving and actuating one or more syringes 9 to dispense materials into the containers 15 and to aspirate materials from the containers. The second arm 35 also carries a sensing device 41 for sensing a property (e.g., pH) of the materials being formulated. These various stations and pieces of equipment are described in more detail below.

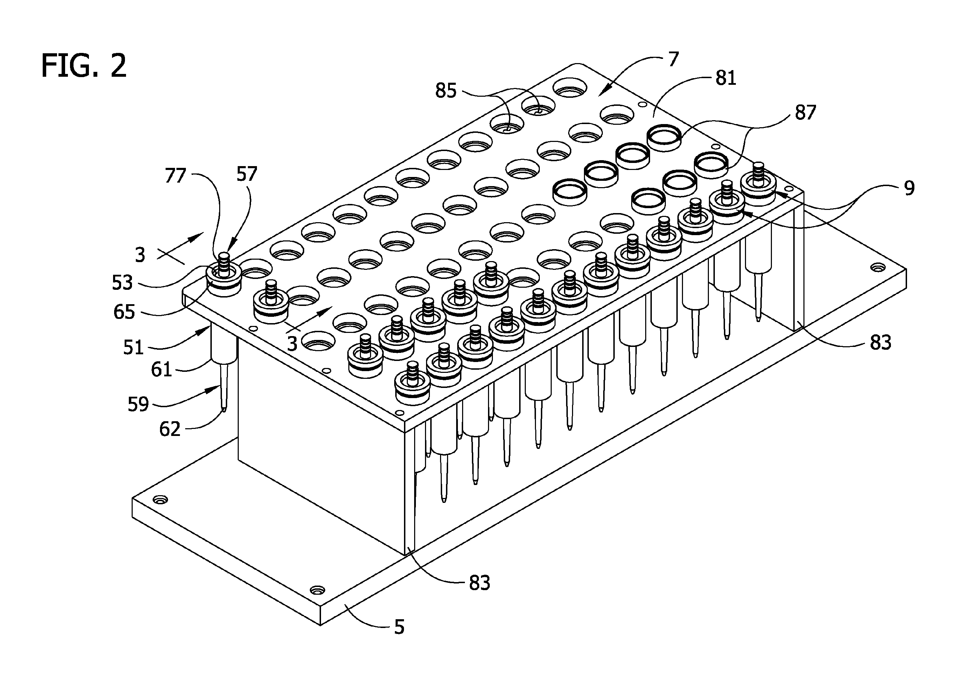

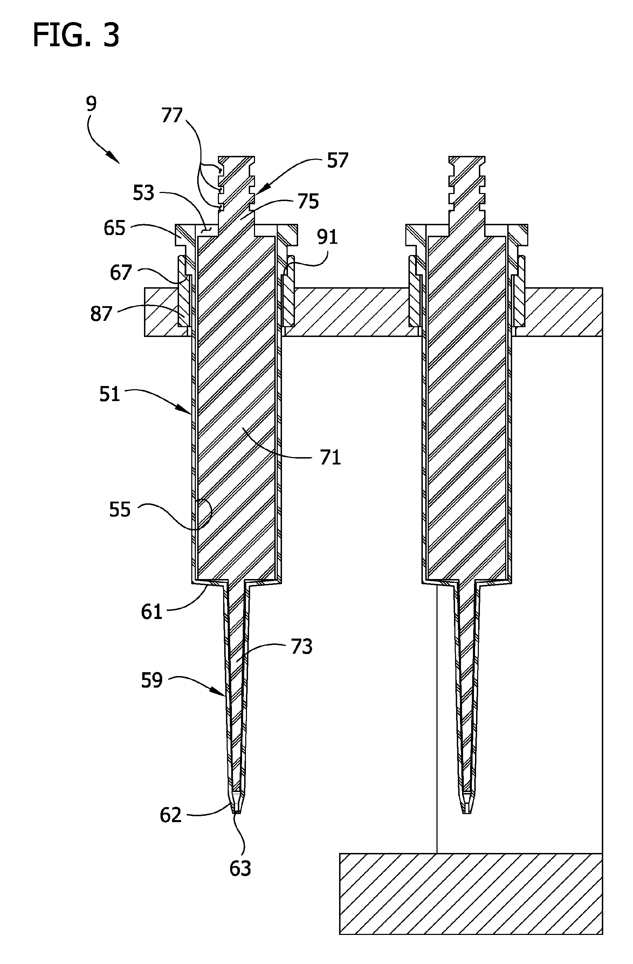

Referring now to Figs. 2 and 3, each syringe 9 comprises a cylinder 51 having an open upper end 53 and defining a volume 55 for holding a material to be dispensed and/or aspirated, a plunger 57 movable up and down in the cylinder, and a nozzle 59 extending down from the lower end 61 of the cylinder and terminating in a nozzle tip 62 defining an orifice 63 through which material is dispensed from the cylinder as the plunger moves down in the cylinder and through which material is aspirated from the cylinder as the plunger moves up in the cylinder. The cylinder 51 has a peripheral flange 65 at its upper end 53 and a downward-facing annular shoulder 67 spaced below the flange. The plunger 57 comprises a body 71 received in the cylinder 51, a stem 73 projecting down from the body into the nozzle 59, and a neck 75 extending up from the body having a series of circumferential grooves 77 therein. When the plunger 57 is fully depressed, as shown in Figs. 2 & 3, the neck 75 of the plunger extends up above the top 53 of the cylinder 51 so that it may be gripped by the syringe moving and actuating apparatus 37, as will be described. The syringe 9 is configured to meet the needs of the work process to be carried out. By way of example but not limitation, the syringe 9 may have the following characteristics: a maximum dispense volume of about 9 mL; a minimum dispense volume of about 10 µL; a maximum dispense rate of about 0.5 mL/sec; a minimum dispense rate of about10 µL/sec; a maximum dispense and aspiration temperature of about 60 degrees C; and a minimum dispense and aspiration temperature of about15 degrees C. The syringe illustrated in Figs. 2 and 3 is of conventional design, except for its connection to and manner of operation by the robot 27, which will be discussed in detail later herein.

The syringe rack 7 comprises a plate 81 with legs 83 (Fig. 2) for supporting the plate above the deck 3, an array of openings 85 in the plate, and annular syringe supports 87 in the openings for supporting the syringes 9 upright. As shown in Fig. 3, each syringe support 87 has an internal upward-facing shoulder 91 for engagement by the downward-facing external shoulder 67 on the cylinder 51 of a respective syringe 9. The configuration is such that when the two shoulders 67, 91 are in contact, the flange 65 at the upper end 53 of the syringe 9 is spaced above the top of the annular support 87 a distance sufficient to enable the cylinder 51 of the syringe to be gripped by the syringe actuating apparatus 37, as will be described.

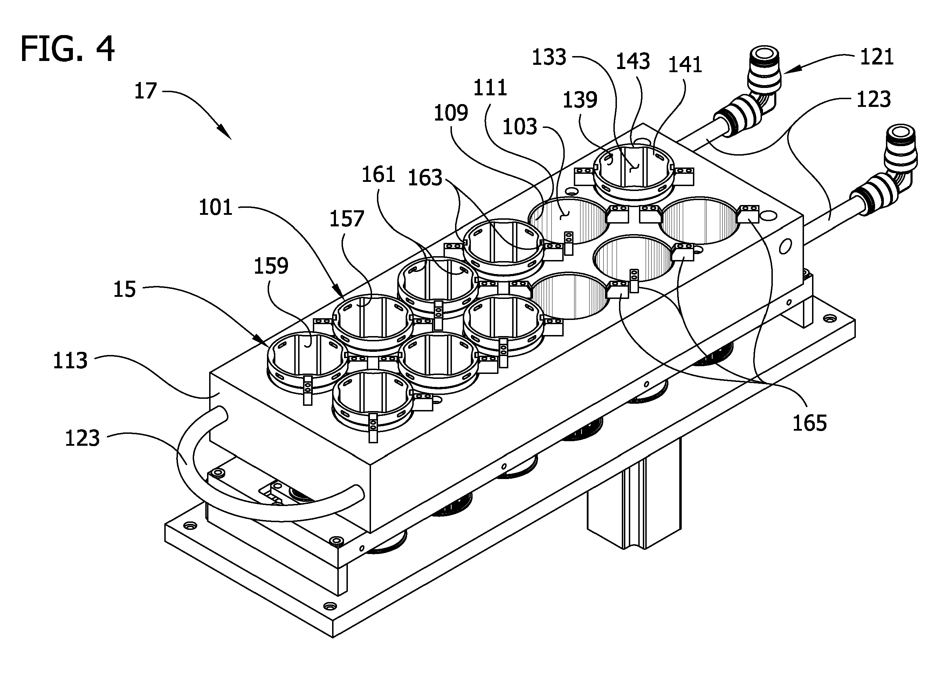

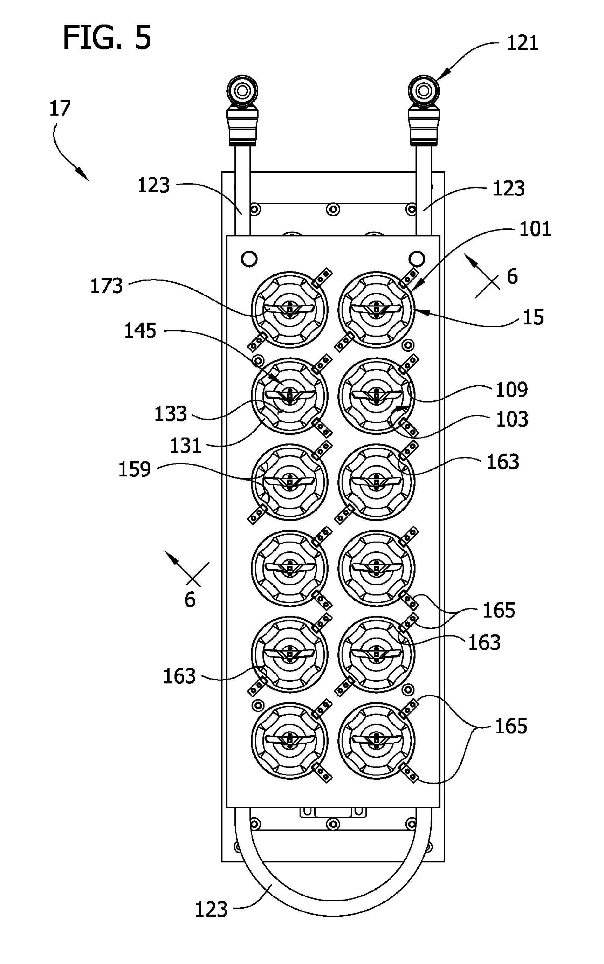

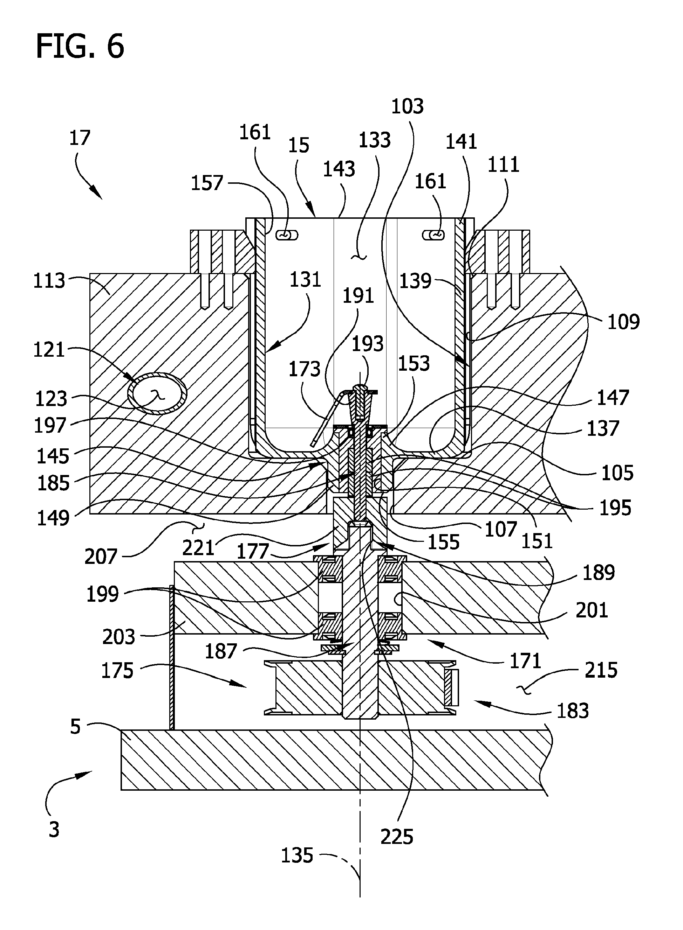

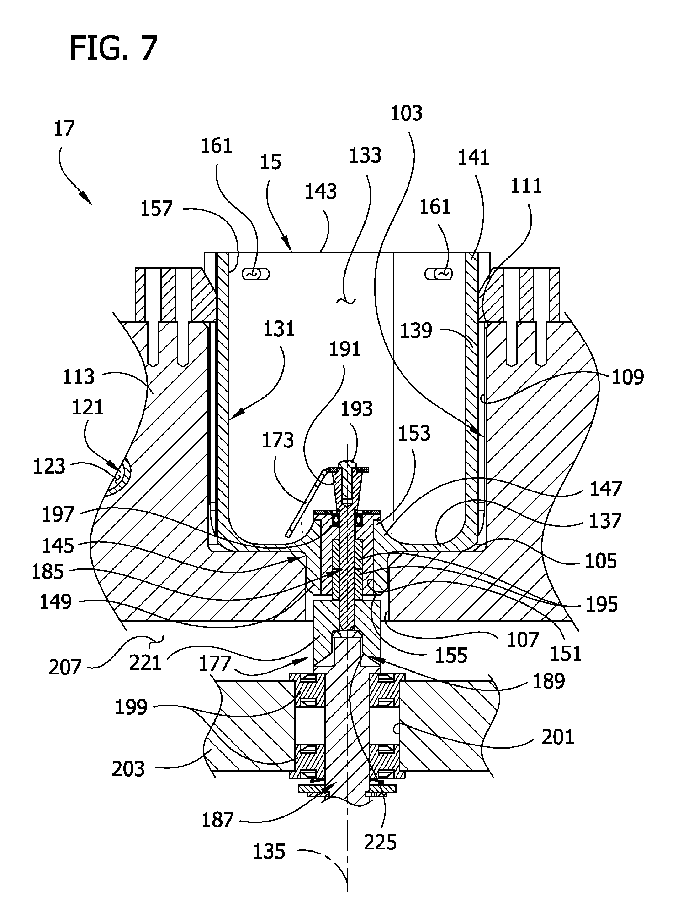

The mixing station 11 is equipped with a set of one or more mixing modules 17 (one of which is illustrated in Figs. 4 and 5) for holding one or more arrays 101 of containers 15. In the embodiment of Fig. 1, two modules 17 are shown, each comprising a block 113 having a 2x6 array 101 of openings or wells 103 therein. Each well 103 has a bottom surface 105 (Figs. 6 & 7), a central bore 107 extending down through the bottom surface, a side surface 109 extending up from the bottom surface, and an open top 111. The well 103 is sized and configured for holding one container 15. The number of modules 17 and the number of wells 103 in each module may vary. By way of example but not limitation, the modules 17 and wells 103 may be configured for holding a plurality of "source" containers which contain suitable source materials for use in making formations, a plurality of "intermediate" containers for holding mixtures of materials in a pre-final condition, and a plurality of "final destination" containers for holding mixtures of materials in a final condition. Preferably, a temperature control system 121 is provided to control the temperature of one or more of the modular blocks 113. In one embodiment (Figs. 4-7), this temperature control system 121 comprises conduits 123 in the modular blocks 113 for the flow of heated or cooled fluid through the blocks. By way of example but not limitation, the temperature control system 121 may have the following parameters: a maximum temperature of about 60 degrees C; a minimum temperature of about 15 degrees C; a heating rate of about 0.5 degrees C/minute; a cooling rate of about 0.5 degrees C/minute; and a thermal uniformity of about +/- 2% of a set point temperature, which may be selectively adjustable to various temperatures in a range including the maximum and minimum temperatures. Other heating and/or cooling systems can be used, including systems which allow the temperature of each container 15 or a group of containers to be controlled independent of other containers or groups of containers.

Referring to Fig. 7, each container 15 comprises a cup-shaped body 131 defining a volume 133 for holding material, a generally vertical central axis 135, a bottom wall 137, an upstanding generally cylindrical side wall 139, and an upper rim 141 defining an open upper end 143 of the container. By way of example but not limitation, the container 15 may have a maximum capacity ranging from about 50-200 ml, such as about 100 ml. The bottom wall 137 of the container 15 includes a central hub 145 having an upper portion 147 which extends up into the interior volume 133 of the container and a lower portion 149 which projects down into the bore 107 in the bottom surface 105 of the well 103 when the container is seated in its respective well. A bore 151 co-axial with the central axis 135 of the body 131 extends through the hub 145 from its upper end 153 to its lower end 155. The inner surface 157 of the container side wall 139 comprises one or more vertical ribs 159 (Fig. 5) which project inward toward the central axis 135 of the container 15 and facilitate achieving a more uniform mixing of the contents of the container. A number of openings 161 (Figs. 6 & 7) are spaced around the sidewall 139 of the container 15 and extend in a radial direction through the rim 141 of the container to allow the container to be gripped and moved by the robot 27, as will appear. Further, the rim 141 is formed with vertical slots 163 which function as keyways that receive keys 165 affixed to the modular blocks 113 to maintain the containers 15 in proper registration (i.e., angular orientation) with respect to their respective wells 103. This registration and its significance will be discussed in more detail later herein.

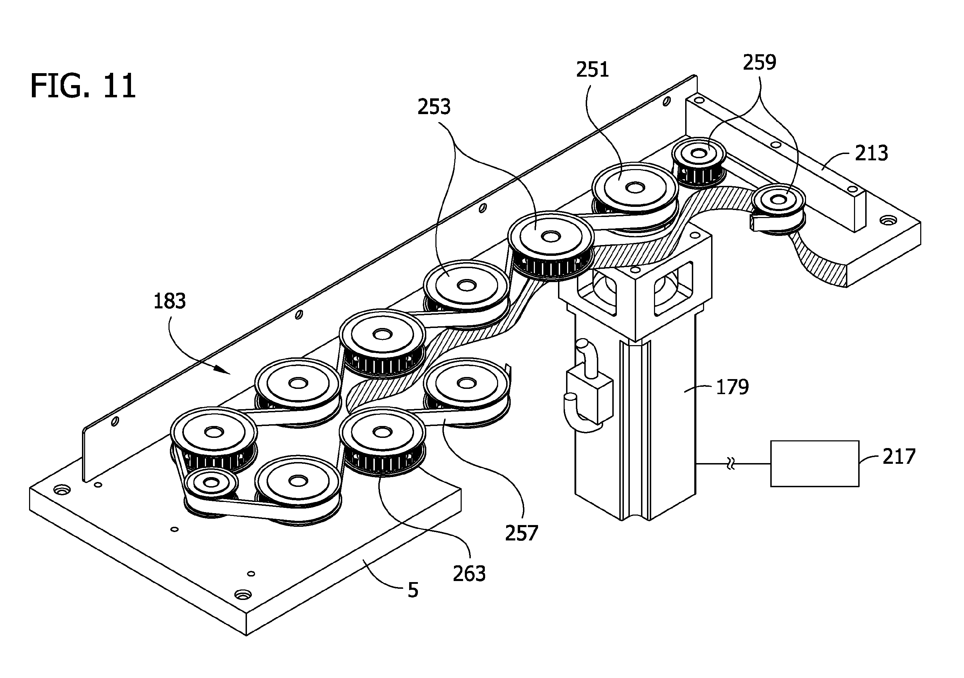

The mixing station 11 is also equipped with apparatus 171 for mixing the contents of the containers 15 (see Figs. 6-11). In particular, this apparatus 171 includes mixing blades 173 in the containers 15 and a drive system 175 associated with each 6x2 array 101 of containers for rotating the blades in the containers. The drive system 175 comprises a number of direct drive mechanisms 177, one for each container 15, extending up through the bottom walls 137 of the containers for rotating the mixing blades 173, a motor 179 (Fig. 11) secured to the underside of the deck 3 and having an output shaft (not shown), and a belt-and-pulley system 183 coupling the motor to the direct drive mechanisms for rotating the mixing blades.

As shown in Fig. 6, each direct drive mechanism 177 includes a mixing shaft 185 extending up through the hub 145 in the container 15, a drive shaft 187 for rotating the mixing shaft, and a releasable coupling 189 for releasably connecting the drive shaft and the mixing shaft. The upper end 191 of the mixing shaft 185 projects above the hub 145 into the interior volume 133 of the container 15. The mixing blade or blades 173 are secured to the mixing shaft 185 adjacent the upper end 191 of the shaft by one or more fasteners 193 for convenient removal and replacement of the blades. The configuration of the blade(s) will vary, depending on the materials being mixing, the mixing speed and other factors known to those skilled in this art.

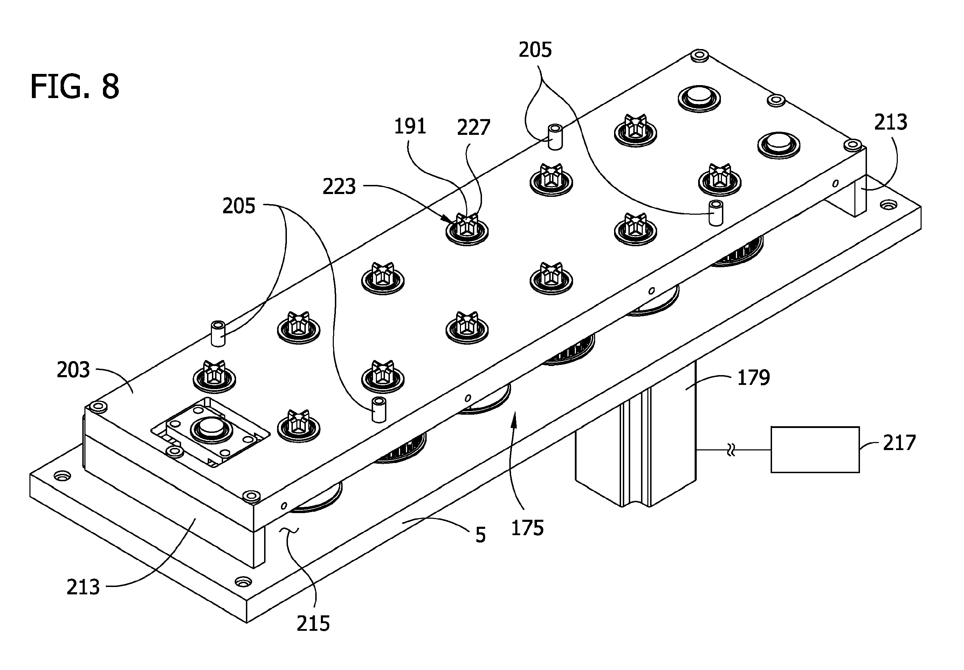

The mixing shaft 185 of each direct drive mechanism 177 rotates in one or more bearings 195 mounted in the bore 151 of the hub 145 of a respective container 15 (Fig. 7). A seal 197 is provided at the upper end of the bore 151 to prevent materials in the container from leaking out of the container. The drive shaft 187 of each direct drive mechanism 177 rotates in a pair of bearings 199 mounted in an opening 201 in a bearing plate 203 positioned between the bottom of the block 113 holding the array 101 of containers 15 and the deck 3 of the work station 1. The bottom of the block 113 is spaced from the bearing plate 203 by spacer members 205 (Fig. 8) to thermally isolate the block from the bearing plate. The spacing maintained by the spacers 205 also provide a vertical space or gap 207 sufficient to accommodate the couplings 189 between the drive shafts 187 and the mixing shafts 185. The bearing plate 203 is spaced above the deck 3 by legs 213 to provide a vertical space or gap 215 sufficient to accommodate the belt-and-pulley system 183.

The coupling 189 between each drive shaft 187 and mixing shaft 185 is illustrated in Figs. 6, 7 and 8. The coupling 189 comprises an upper coupling member 221 on the lower end of the mixing shaft 185 and a lower coupling member 223 on the upper end of the drive shaft 187. In this particular embodiment, the upper coupling member 221 comprises a downward opening slot formation 225 (e.g., a cross-shaped slot formation) and the lower coupling member 223 comprises an upward projecting rib formation 227 (e.g., a cross-shaped rib formation, e.g., see Fig. 8). The rib formation 227 is configured for reception in the slot formation 225 of the upper coupling member 221 such that relative rotation between the two shafts 185, 187 is substantially prevented when the two coupling members are engaged, as during a mixing operation. The coupling 189 allows axial (e.g., vertical) separation of the two shafts 185, 187 so that the container 15, mixing shaft, upper coupling member 221, and mixing blade(s) 173 can be removed as a unit from the well 103 of the mixing module 17 for transport of the container by the robot 27 to a different location at the workstation 1. Thereafter, the container 15 can be replaced robotically in the well 103 and the coupling members 221, 223 re-engaged for another mixing operation. Other releasable couplings between the mixing and drive shafts 185, 187 can be used without departing from the scope of this invention.

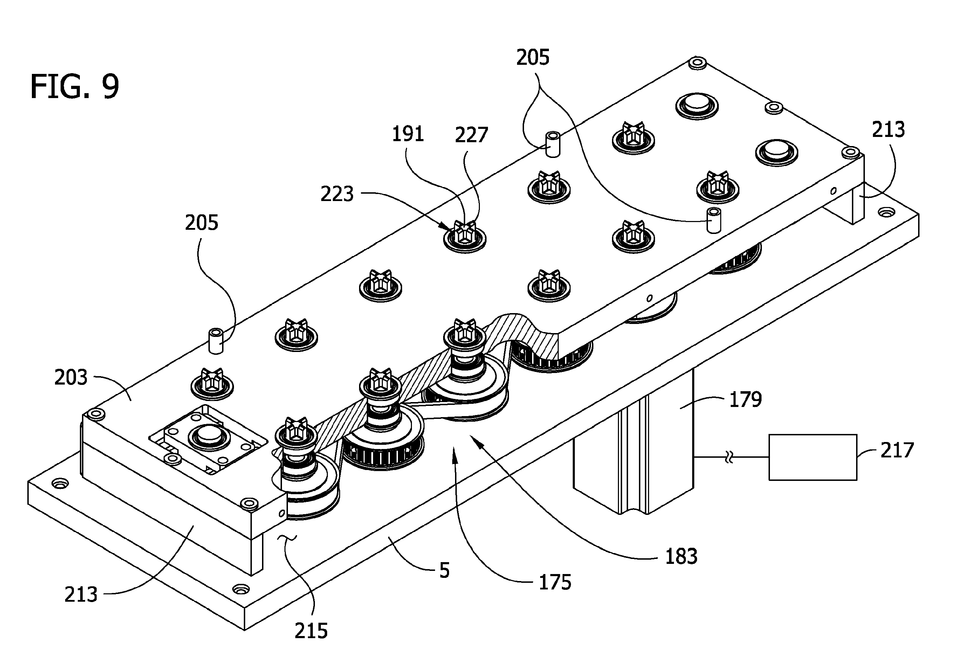

Figs. 9-11 illustrate the belt-and-pulley system 183 for rotating the various drive shafts 187 associated with one of the modular blocks 113 at the mixing station 11. The system 183 comprises a master drive pulley 251 mounted on one of the drive shafts 187 and slave pulleys 253 mounted on the other drive shafts. The output shaft 181 of the motor 179 is connected by a coupling (not shown) to the drive shaft 187 mounting the master pulley 251. The system 183 also includes a belt 257 trained around the master pulley 251, the slave pulleys 253 and several idler pulleys 259 such that rotation of the motor output shaft 181 rotates all of the drive shafts, mixing shafts 185 and mixing blades 173 to effect mixing of the materials in the containers 15. The speeds of rotation of the mixing blades 173 will vary (e.g., up to 2000 rpm). Further, the system 183 is suitably capable of mixing materials having a viscosity of up to at least about 2000 cps, and preferably up to about 25,000 cps.

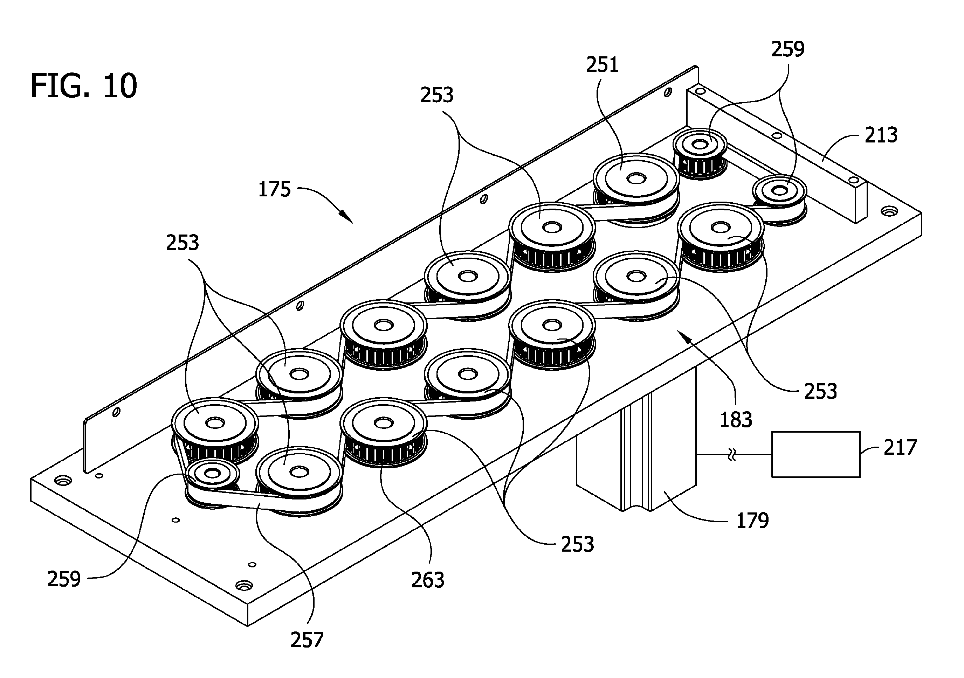

As best understood in reference to Fig. 10, the belt 257 of the illustrated belt and pulley system 183 weaves back and forth between the pulleys 251, 253 in such a way that the pulleys in each row are rotated by the belt in a direction that is opposite the direction of rotation of the neighboring pulleys in the same row. Further, the belt 257 and pulleys 251, 253 are arranged so that each pulley in one row is rotated in the same direction as the neighboring pulley in the adjacent row. This reduces the number of idler pulleys that are needed and provides a compact belt and pulley system 183.

Because the pulleys 251, 253 do not all rotate in the same direction, some of the mixing blades are 173 rotated in a counterclockwise direction while other mixing blades are rotated in a clockwise direction. Mixing blades 173 that are rotated counterclockwise are suitably mirror images of the mixing blades that are rotated clockwise. This reduces the likelihood that the direction of rotation will affect the mixing of the materials in the container. For example, the mixing blades 173 suitably have pitched blades that circulate the material in the container 15 so that material in the center of the container moves upwardly (or downwardly) while material along the periphery of the container moves in the opposite direction. Variation in the mixing action that is produced in the containers 15 by the mixing blades 173 can be reduced by using counterclockwise rotating mixing blades that are mirror images of the clockwise rotating mixing blades. Thus, the pitch of the mixing blades 173 corresponds to the direction of rotation so that the mixing by the counterclockwise rotating blades is equivalent to the mixing by the clockwise rotating blades. Other belt and pulley systems and other types of drive systems may be used without departing from the scope of the invention.

As noted above, the coupling 189 allows a container 15, its mixing shaft 185, and its mixing blade 173 to be loaded and unloaded from the mixing module as a unit. The slots 163 and keys 165 for the containers 15 having mirror image mixing blades 173 and the slots and keys for the other containers are suitably arranged in geometric configurations that are mutually incompatible. This arrangement prevents a container 15 having a mixing blade 173 designed to be rotated in a counterclockwise direction from being inadvertently loaded into a well 103 that is associated with a pulley 251, 253 driven by the belt 257 in a clockwise direction (and vice-versa). In the embodiment illustrated in Figs. 4 and 5, for example, the slots 163 and keys 165 of containers 15 having mixing blades 173 to be rotated in a first direction are spaced from one another circumferentially around the rim 141 of the respective container a first amount (e.g., about 180 degrees), while the slots and keys of containers having mixing blades to be rotated in the opposite direction are spaced from one another circumferentially around the rim a different amount (e.g., about 90 degrees). Attempts to load a container 15 having slots that are spaced about 180 degrees from one another into a well 103 having keys 165 that are spaced only about 90 degrees from one another will be thwarted by the incompatible geometric arrangements of the slots and keys (and vice-versa).

Desirably, the belt 257 is a timing belt having teeth (not shown) which engage complementary teeth 263 on the pulleys so that rotation of the drive and mixing shafts 185, 187 can be maintained in timed relation and fixed angular orientation relative to one another. The master and slave pulleys 251, 253 are secured in fixed position to respective drive shafts 187 by set screws (not shown) or other suitable means which can be loosened to allow adjustment of the relative angular positions of the shafts, and then re-tightened. In one embodiment, the motor 179 is an AC servomotor controlled by a suitable controller 217 to rotate the direct drive mechanisms 177 and mixing blades 173 at the same selected mixing speed and in synchronization (see Fig. 5). Further, at the end of a mixing interval, the controller 217 is programmed to operate the motor 179 to move all of the direct drive mechanisms 177 to a predetermined "home" position (see e.g., Fig. 5) in which the mixing blades 173 in the containers 15 reside in known rotary positions to provide an unobstructed path extending from the open top 143 of a container down past the mixing blade(s) to the bottom wall 137 of the container. In this manner, the robot 27 can be operated to move a syringe 9 down along this unobstructed path to an aspirating position adjacent the bottom wall of the container, taking into account the predetermined rotary position of the mixing blade(s) in the container so that there is no contact between the blade and the syringe.

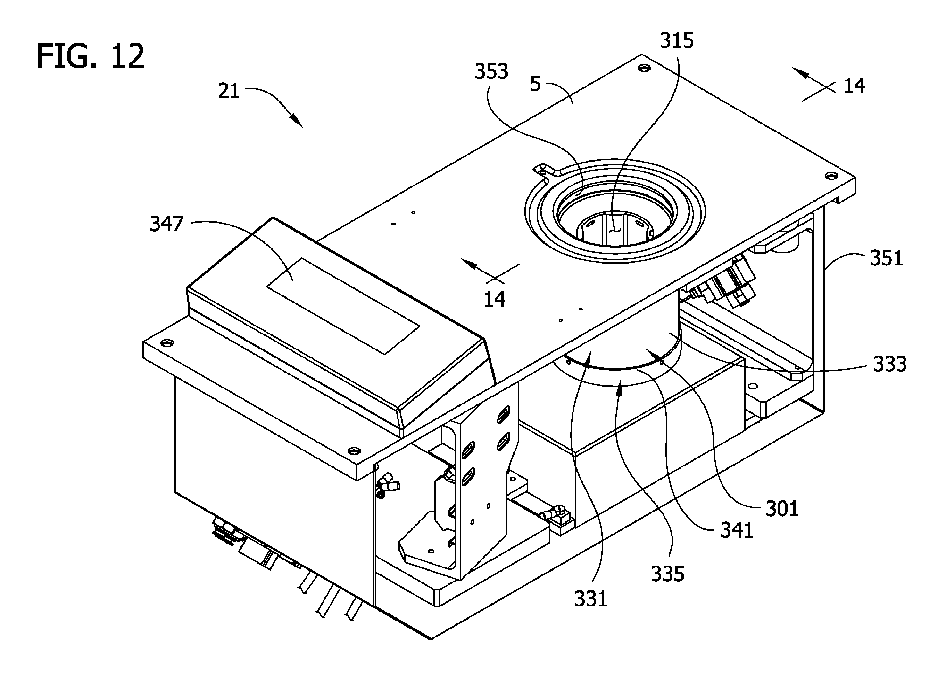

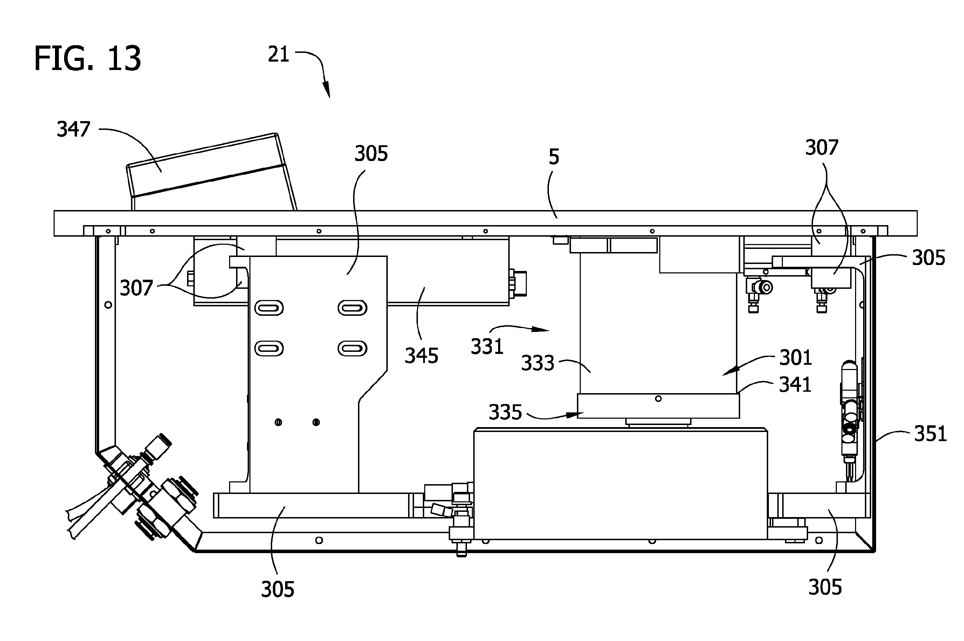

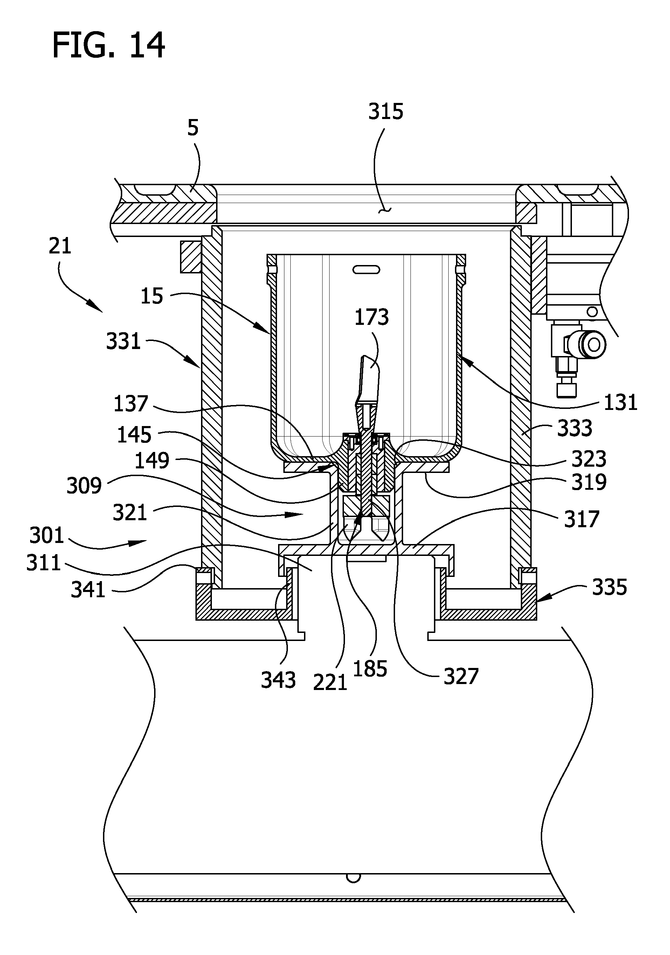

The weighing apparatus 21 at the weigh station 19 comprises a weigh cell 301 mounted on a base plate 303 secured by brackets 305 to the underside of one of the deck panels 5 (see Figs. 12-14). Rubber spacers 307 (Fig. 13) between the brackets 305 and the deck 3 function to reduce the transmission of vibration from the deck panel 5 to the base plate 303 and weigh cell 301. A container support 309 is positioned on a pedestal 311 of the weigh cell 301 directly below an opening 315 in the deck panel. The support 305 comprises a base 317 which rests on the pedestal 311 of the weigh cell 301, a platform 319 above the base for supporting the bottom wall 137 of a container 15, and a tubular column 321 between the base and the platform. The column 321 has an open upper end 323 and defines a vertical space 325 for receiving the lower portion 149 of the hub 145, the lower end 327 of the mixing shaft 185 and the upper coupling member221. The support 309 and a container 15 on the support are surrounded by an enclosure 331 comprising a cylindrical shield 333 attached at its upper end to the deck 3, and a spill pan 335 at the lower end of the shield for catching any fluids which might spill down into the space 337 defined by the shield. The spill pan 335 has an outer rim 341 fastened to the shield 333 and an inner rim 343 which surrounds the pedestal 311 of the weigh cell 301. The weighing apparatus 21 also includes an electronics module 345 (Fig. 13) located on the underside of the deck panel 5 and a display 347 on top of the deck panel. The parts of the weighing apparatus 21 located below the deck panel 5 are enclosed by a housing 351 (Fig. 12) attached to the deck panel. When the weighing apparatus 21 is in use, the opening 315 in the deck panel 5 is closed by a cover 353 which is pneumatically moved between open and closed positions.

The robot 27 shown in Fig. 1 is a 3-axis programmable robot capable of moving objects along X, Y and Z axes, the X and Y axes typically being generally horizontal and the Z axis generally vertical. As a result, an object can be moved in a predetermined manner to essentially any X/Y/Z position within the working range of the robot 27. For convenience, dimensions and directions along the X, Y and Z axes shown in Fig. 1 will be referred to herein as "X" dimensions/directions, "Y" dimensions/directions and "Z" dimensions/directions, respectively.

The robot 27 includes the aforementioned first and second arms 31, 35 movable along a horizontal track 401 corresponding to the X axis. (The number of arms may vary, e.g., one, two or three.) A first vertical Z-member 403 comprising a rack 405 is mounted on the first arm 331 for movement along the arm in a Y-direction and for up-and-down vertical movement relative to the arm in a Z-direction. Similarly, a second vertical Z-member 407 comprising a rack 409 is mounted on the second arm 35 for movement in Y and Z directions. The arms 31, 35 and Z-members 403, 407 are moved in a conventional manner. Users control the robot 27 using a robotic control system (not shown), which typically includes software for both protocol development and execution.
Software useful in the robotic control system is Renaissance Impressionist® and Epoch® software, available from Symyx Technologies, Inc. (Santa Clara, California). Renaissance Impressionist® Software is a general laboratory automation package for creating and executing laboratory procedures.

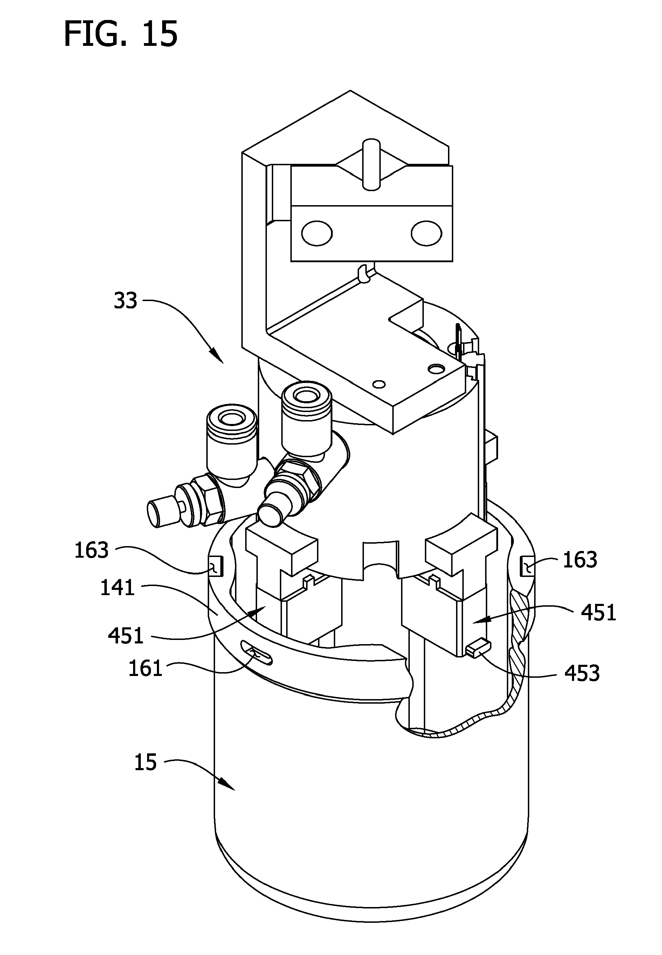

The gripping mechanism 33 is carried by the first arm 31 and is attached by a bracket 421 to the lower end of the vertically movable rack 405. The gripping mechanism 33 comprises a plurality of grippers 451 (e.g., three or four grippers are shown in Fig. 15) having outward projecting pins 453 sized and configured for reception in the openings 161 in the rim 141 of a container 15. The grippers 451 are operated by suitable means (e.g., pneumatics) to move between a non-gripping or release position in which the grippers are contracted radially inward for entry into and exit from a container 15 via its open upper end 143, and a gripping position in which the grippers are extended radially outward for insertion of the pins 453 on the grippers into respective openings 161 in the container to grip it. The container 15 and gripping mechanism 33 are moved by the robot 27 in X, Y and Z directions in a predetermined (programmed) manner. The gripping mechanism may have other configurations without departing from the scope of this invention.

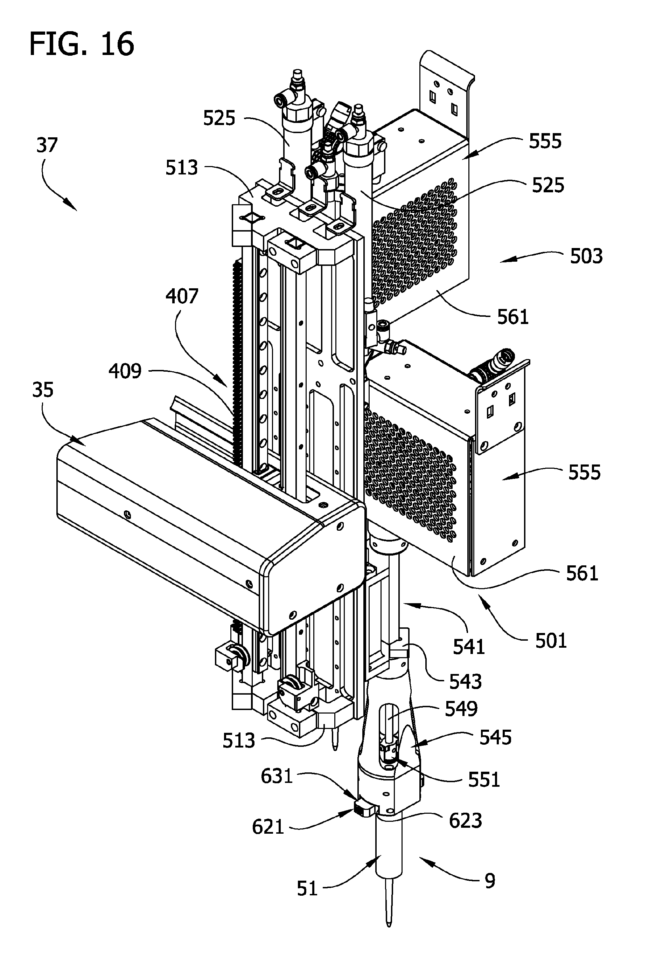

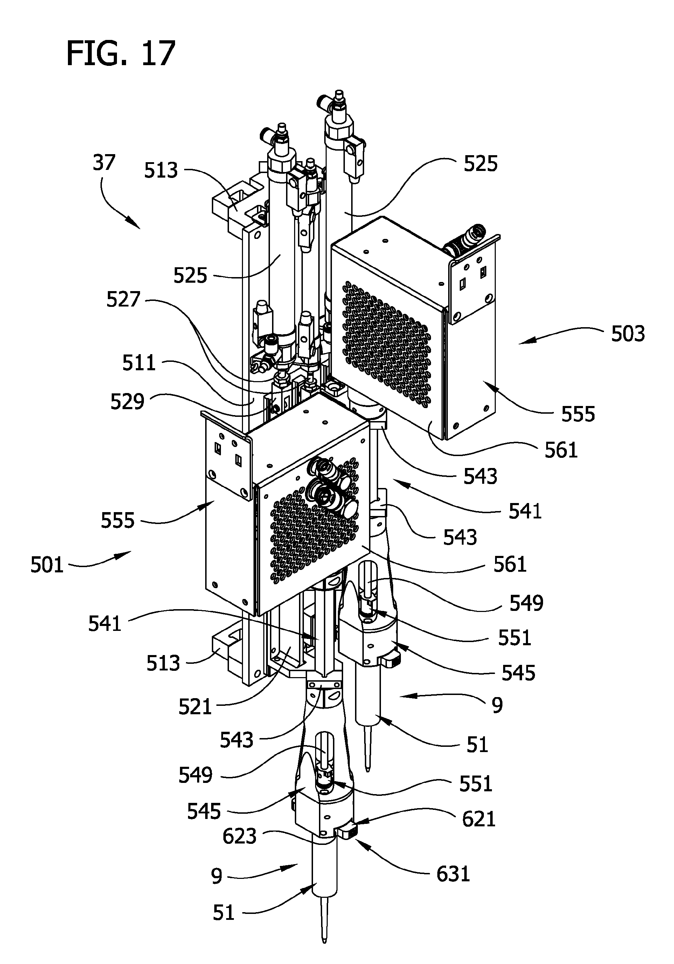

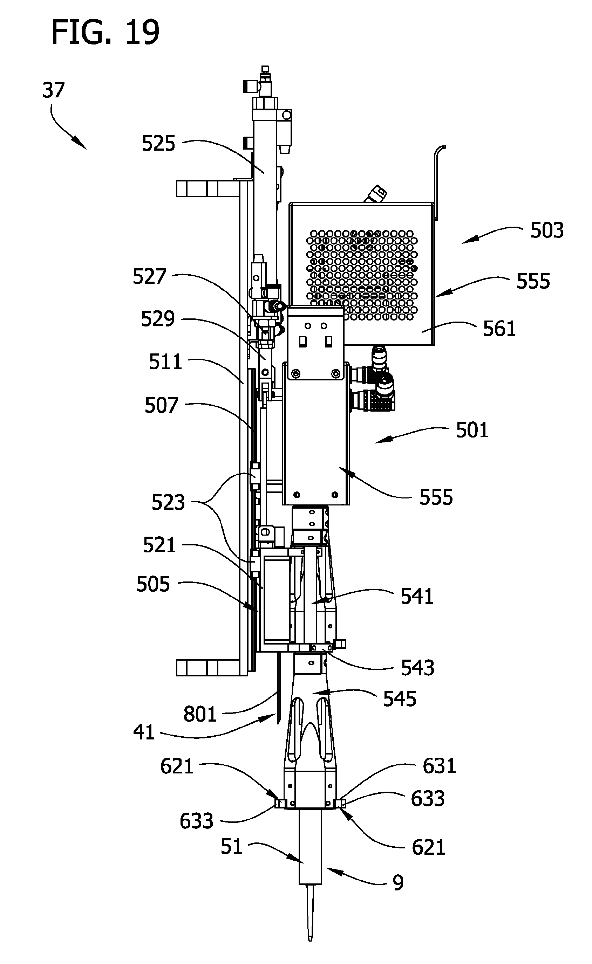

Figs. 16-22 illustrate apparatus 37 on the second arm 35 of the robot 27 for actuating one or more syringes 9 to dispense materials into the containers 15 and to aspirate materials from the containers. In this particular embodiment, the apparatus 37 comprises a first device, generally designated 501, for actuating a first syringe 9 and a second device, generally designated 503, for actuating a second syringe. It will be understood that the number of syringe actuating devices on the robot 27 can vary from one to two or more. Each device 501, 503 is mounted on its own carriage 505 which slides up and down on a vertical rail 507 on a mounting plate 511 secured by brackets 513 to the Z-member 407 on the second arm 35. In one embodiment, the carriage 505 comprises a bracket 521 and a pair of vertically spaced sliders 523 fastened to the bracket for sliding engagement with the rail 507. The carriages 505 are moved up and down on their respective rails 507 by a pair of linear actuators 525, e.g., pneumatic cylinders secured to the mounting plate 511. The cylinders 525 have piston rods 527 with clevis connections 529 to respective carriages 505 such that retraction of the rods causes the carriages 505 to slide up on the rails 507 and extension of the rods causes the carriages to slide down on the rails. The range of vertical movement of each device 501, 503 along the Z-axis is thus the combined range of vertical travel of the rack 409 on the second arm 35 and the vertical travel of the carriage 505 on the rail 507 , the latter range corresponding to the stroke of the cylinder 525 moving the carriage. Desirably, the cylinders 525 are operable to move the carriages 505 independently of one another.

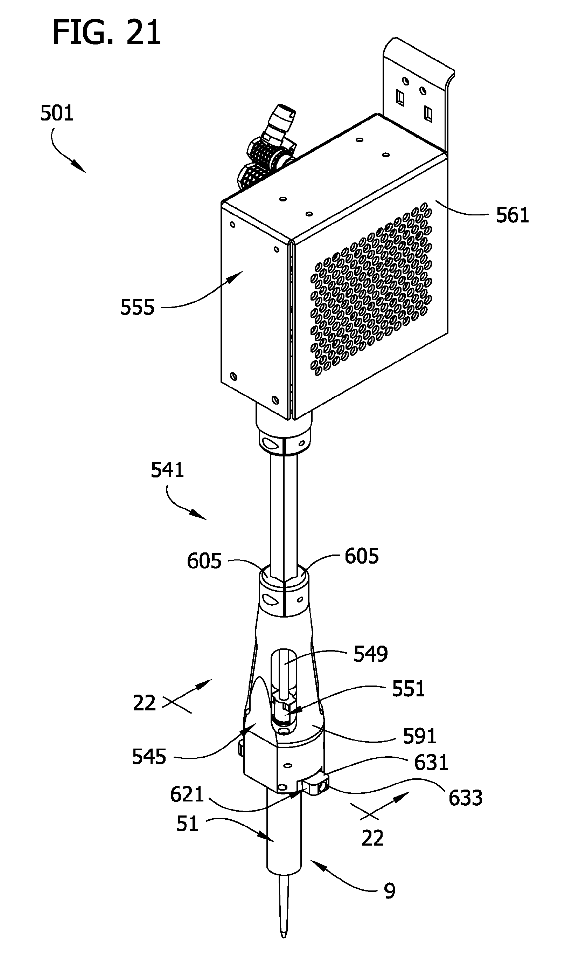

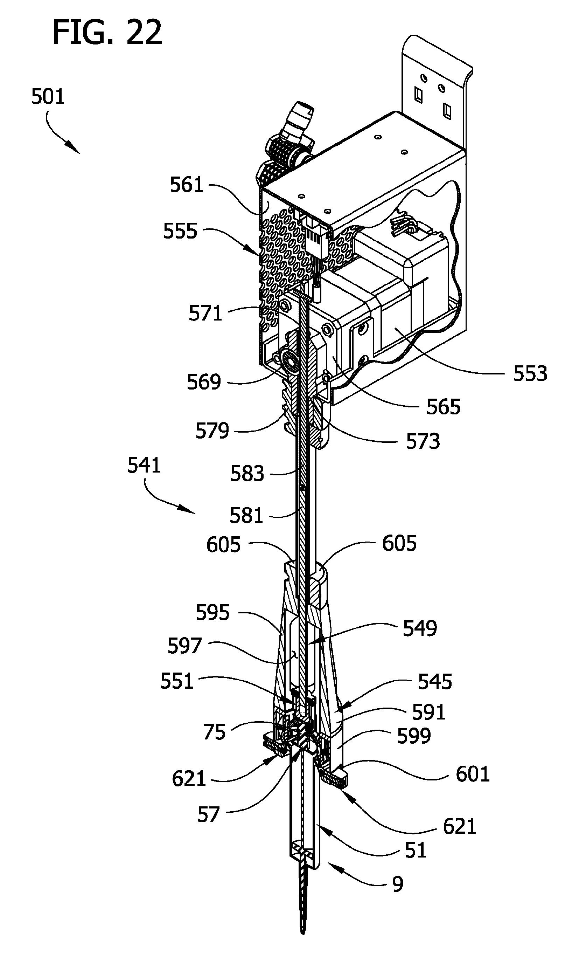

The two syringe actuating devices 501, 503 are of substantially the same construction and corresponding parts are designated by the same reference numbers. In particular, and referring to Figs. 21 and 22, each device 501, 503 comprises a tubular support 541 held by the bracket 521 of a respective carriage 505 in a generally vertical position, the support being attached to the bracket by clamps 543 fastened to upper and lower arms of the bracket. The devices 501, 503 also include a holder 545 secured to the lower end of the tubular support 541 for holding the cylinder 51 of a syringe 9, an elongate actuator 549 movable in the Z direction relative to the holder, and a coupling, generally designated 551, for coupling the actuator 549 to the plunger 57 of the syringe. The actuator 549 is moved up and down in the Z-direction by means including a stepper motor 553 in an enclosure 555 at the upper end of the tubular support 541. As will be described, the arrangement is such that when the cylinder 51 of the syringe 9 is held by the holder 545, movement of the actuator 549 in a first direction (upward) causes the plunger 57 to move up in the cylinder and movement of the actuator in a second direction (downward) causes the plunger to move down in the cylinder. The components of the two syringe actuating devices 501, 503 are described in more detail below.

Referring to Fig. 22, the motor enclosure 555 of each of the devices 501, 503 comprises a rigid bottom wall 559 for supporting the motor 553 and at least one perforated side wall 561 to facilitate cooling of the motor. The stepper motor 553 has an output shaft (not shown) which drives a gear reduction mechanism 565 to rotate a pinion gear 569 in mesh with teeth 571 on an upper section 583 of the actuator 549 to move the actuator up and down in a controlled manner. The upper section 583 of the actuator 549 is movable in a guide 573 affixed to the motor enclosure 555 and to the upper end of the tubular support 541 by a coupling 579. The actuator 549 extends down through the guide 573 into the tubular support 541. The actuator 549 has a lower section 581, the upper end of which is pin-connected to the upper actuator section 583 and the lower end of which is connected to the coupling 551 in a manner to be described.

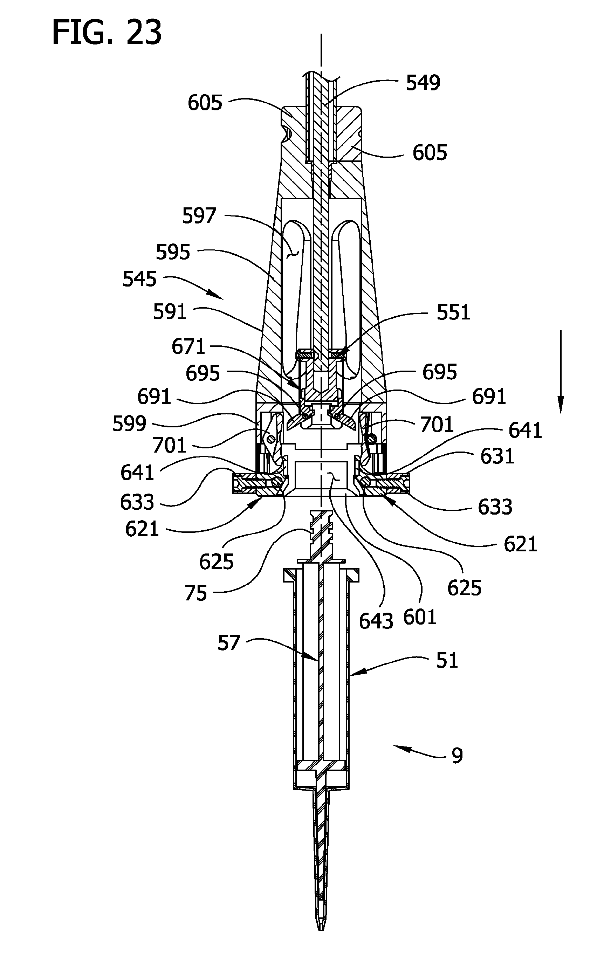

The holder 545 comprises a bell-shaped housing 591 (Figs. 22 and 23) having a central vertical axis generally coincident with the vertical axis of the actuator 549. The housing 591 has an upper tapered 595 section formed with a central cavity 597 and a lower section 599 comprising an annular skirt 601 which defines an open lower end of the housing. The upper section 595 includes opposing clamping portions 605 connected by fasteners (not shown) which, when tightened, clamp the two clamping portions against the tubular support 541 to secure the housing 591 to the support. The actuator 549 extends down through the tubular support 541 and into the cavity 597 of the housing 591. The coupling 551 attached to the lower end of the actuator 549 is positioned inside the housing 591 in the central cavity 597.

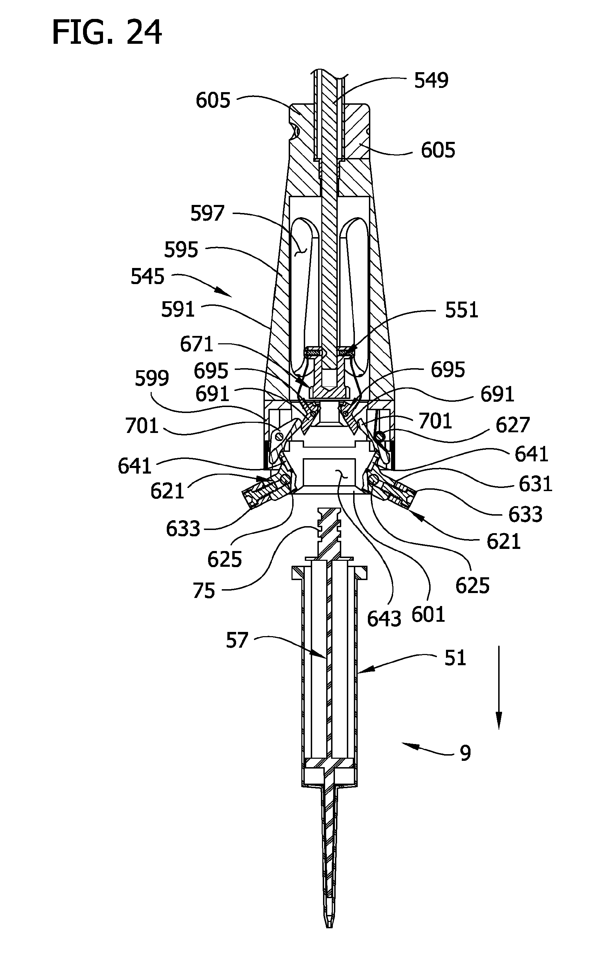

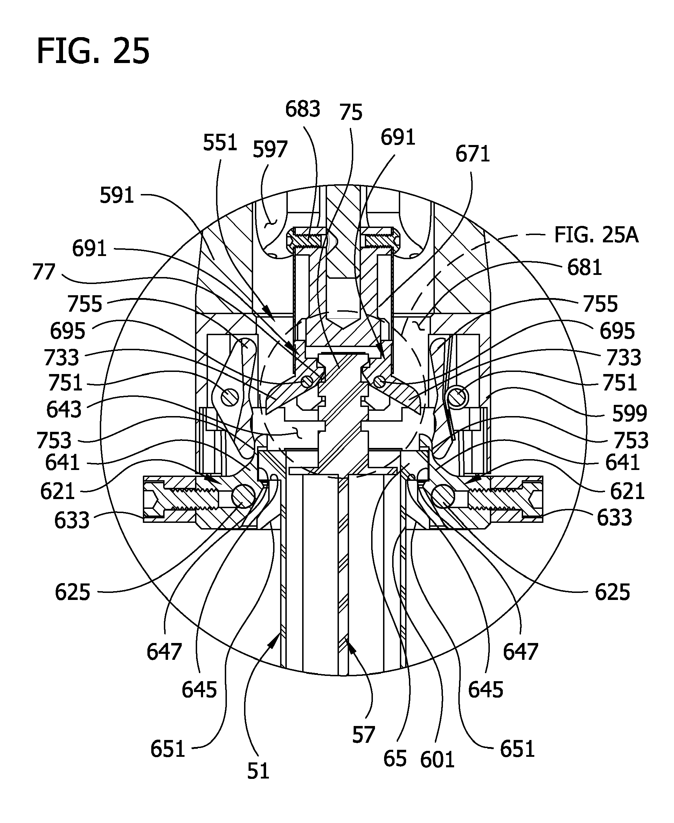

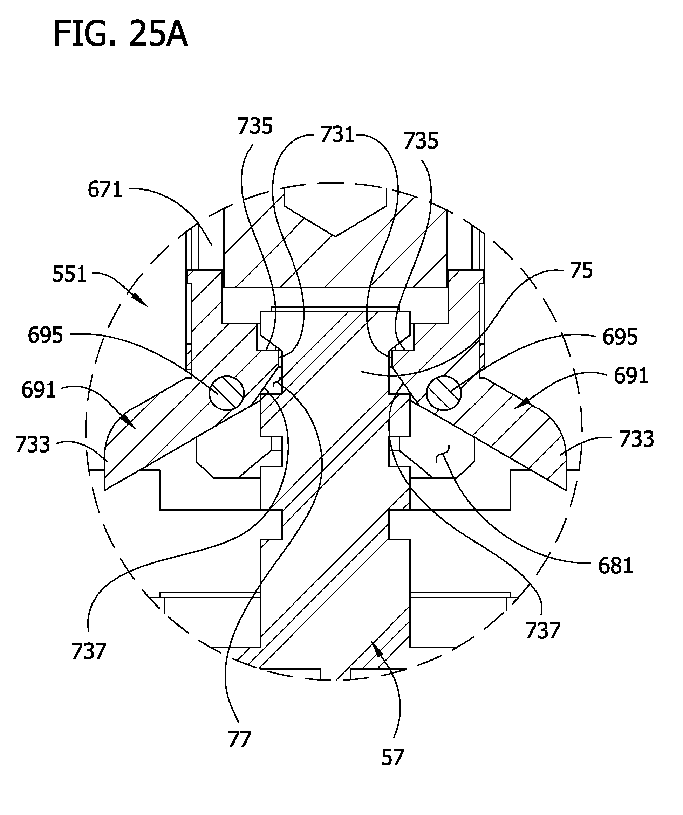

The open lower end of the housing 591 permits a syringe 9 to enter the housing to a position to be held by the holder 545 and later to exit the housing upon release of the syringe by the holder. In this regard, the holder 545 includes at least one and preferably two or more holding members 621 mounted on the housing 591 for movement between a holding position for holding a syringe 9 and a release position for releasing the syringe. In the embodiment of Figs. 24 and 25, two holding members 621 are spaced apart directly opposite one another for receiving a syringe 9 between them. Each holding member 621 is positioned in a notch 623 in the skirt 601 of the housing 591 and pivots on a pin 625 bridging the notch about a horizontal axis from the stated holding position shown in Fig. 23 to the stated release position shown in Fig. 24. The holding members 621 are biased by springs 627 toward their holding positions and can be moved against the bias of the springs to their release position by a release mechanism 631. In the illustrated embodiment, the release mechanism 631 comprises release members 633 fastened to the outer ends of the holding members 621. These release members 633 project laterally outward beyond the skirt 601 of the housing 591 and are adapted to be actuated (e.g., pushed down) to pivot the holding members 621 to their release positions.

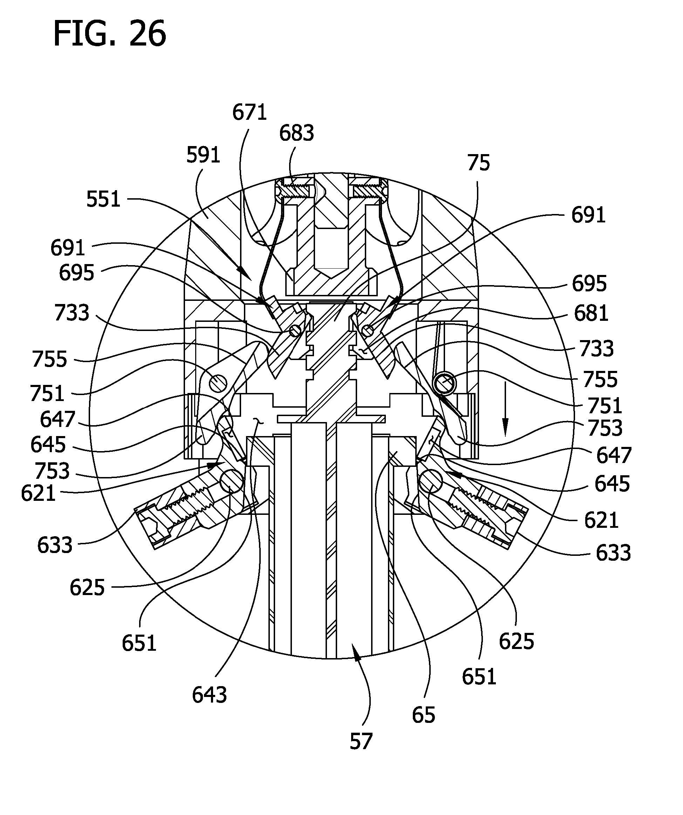

As best illustrated in Figs. 23 and 25, the holding members 621 have opposing inner ends 641 spaced from one another to define an opening 643 for receiving the cylinder 51 of a syringe 9. The inner ends 641 are configured to have upward-facing shoulders 645 which are adapted to underlie the flange 65 of a syringe 9 to support the syringe against downward movement relative to the holder 645 when the holding members 621 are in their holding positions. In a particularly desirable embodiment, the inner ends 641 of the holding members 621 are formed with recesses 647 (defined in part by the upward-facing shoulders 645) for receiving the flange 65 of the syringe 9 so that the syringe is also held against substantial upward movement relative to the holder 545. The inner ends 641 of the holding members 621 have lower faces 651 below the shoulders 645 which slope down and away from the syringe cylinder 51. These faces 651 are configured for contact by the flange 65 on the cylinder 51 when the syringe 9 is loaded into the holder 545, i.e., when the robot 27 moves the holder down so that the cavity 597 begins to receive the syringe. This contact causes the holding members 621 to pivot against the bias of the springs 627 toward their release positions (Figs. 24 and 26). As the robot 27 continues to move the holder 545 down, the syringe 9 reaches a level relative to the holder 545 such that the flange 65 is above the shoulders 645 of the holding members 621, thereby allowing the holding members to snap back to their holding positions so that the flange of the syringe is received in the recesses 647 and supported by the shoulders 645. To release the syringe 9 from the holding members 621, the release members 633 at the outer end of the holding member 621 are moved down to rotate the holding members against the urging of the springs 627 to their release positions in which the shoulders 645 of the holding members rise above the flange 65 of the syringe. In this position the lower faces 651 of the holding members 621 are configured to assume a position (e.g., generally vertical as viewed in Fig. 26) in which that they do not interfere with the exit of the syringe 9 from the holder 545 as it drops down through the opening 643 in the housing. The holder housing and holding members described above may have other configurations.

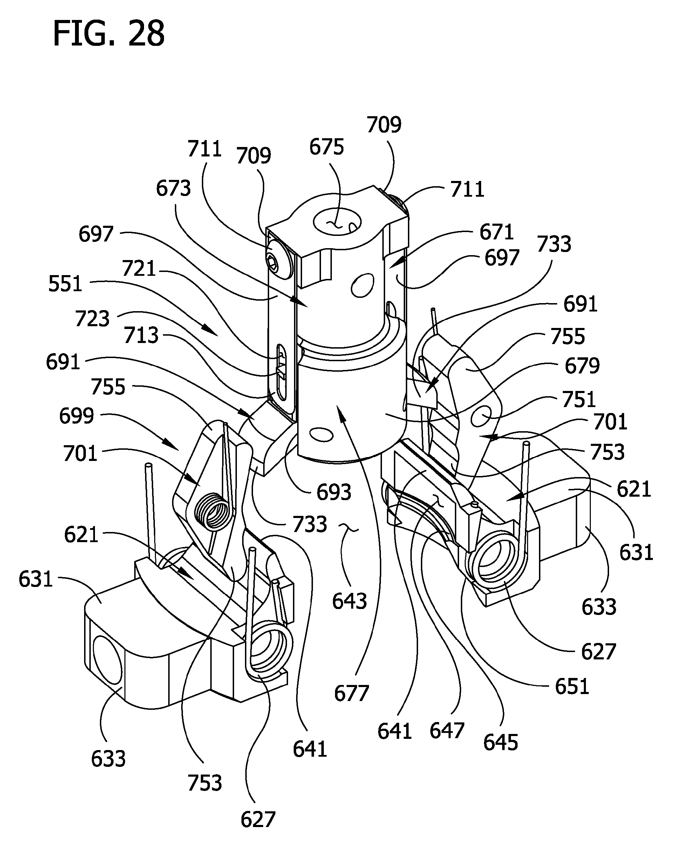

Referring to Fig. 28, the coupling 551 for coupling the movable actuator 549 to the plunger 57 of the syringe 9 comprises a housing 671 located in the cavity 597 of the holder housing 591. The coupling housing 671 has an upper section 673 with a bore 675 for receiving the lower end of the actuator 549 and a lower section 677 having an annular wall 679 defining a chamber 681 for receiving the plunger 57 of a syringe 9. The housing 671 is secured to the actuator 649 by a set screw 683. At least one and preferably two or more coupling members 691 are mounted on the lower section 677 of the coupling housing 671 for movement between a coupling position (Fig. 25) for coupling the actuator 549 to the plunger 57 of the syringe 9 and a de-coupling position for de-coupling the actuator from the syringe (Fig. 26). In one embodiment, two coupling members 691 are positioned directly opposite one another and spaced apart for receiving the plunger 57 of a syringe 9 between them. Each coupling member 691 is positioned in a notch 693 in the annular wall 679 of the coupling housing 671. Each of the coupling members 691 is mounted on a pin 695 bridging the notch 693 for pivoting about a horizontal axis between its coupling and de-coupling positions. The coupling members 693 are biased by springs 697 toward their coupling positions and can be moved against the bias of the springs to their de-coupling positions by a linkage 699 comprising a pair of levers 701, as will be described later. As best shown in Fig. 28, the springs 697 are leaf springs having upper ends 709 secured by fasteners 711 to the upper section of the housing 671 and lower ends 713 which extend down on the outside of the coupling members 691 for urging the coupling members toward their coupling positions. Each spring 697 has a longitudinal slot 721 toward its lower end 713 for receiving a projection 723 on a respective coupling member 691. The projection 723 moves along the slot 721 as the spring 697 deflects in response to pivotal movement of the coupling member 691 between its coupling and de-coupling positions.

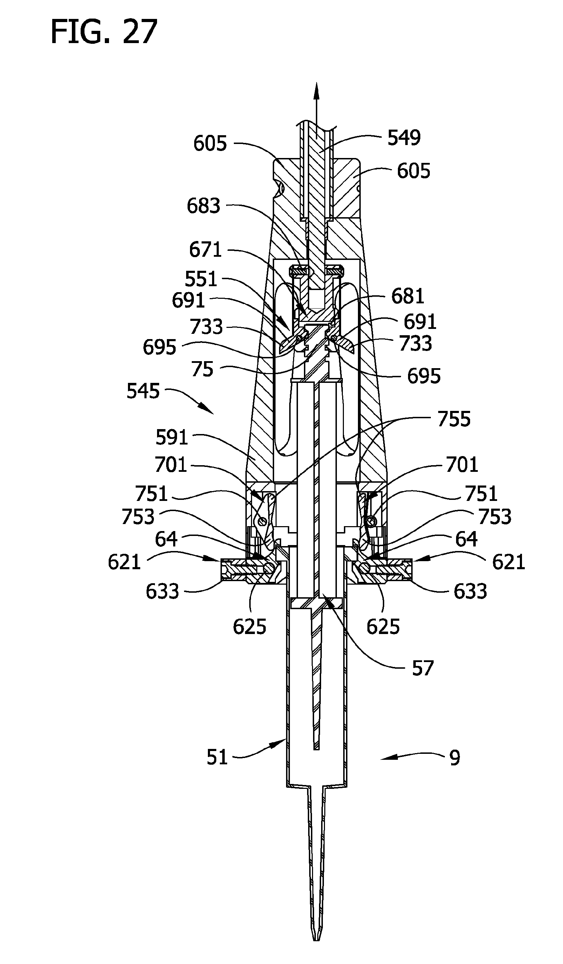

As shown in Fig. 25A, the coupling members 691 have inner ends 731 facing one another and opposite outer ends 733. The inner ends 731 are configured to have upward-facing shoulders 735 which are adapted to be received in the upper groove 77 of a plunger 57 positioned in the chamber 681 of the coupling housing 671 when the coupling members 691 are in their coupling positions. The inner ends 731 of the coupling members 691 also have lower faces 737 below the shoulders 735 which slope down and away from the plunger 57. These faces 737 are configured for contact by the upper end of the plunger 57 of the syringe 9 when the coupler housing 671 is moved down (e.g., by the actuator 549) to a position in which the plunger is received in the chamber 681 of the coupler housing. This contact causes the coupling members 691 to pivot against the bias of the springs 697 toward their de-coupling positions (Fig. 26). When the upper groove 77 in the plunger 57 moves into registry with the shoulders 735 of the coupling members 691, the coupling members snap back to their coupling positions (Fig. 25A) in which the shoulders are received in the groove. In this position, the plunger 57 is securely coupled to the actuator 549 (via the coupling housing 671) so that up and down movement of the actuator causes corresponding movement of the plunger in the cylinder 51 of a syringe 9 held by the holder 545, as shown in Fig. 27. The central cavity 597 of the bell-shaped holder housing 591 has a vertical dimension sufficient to accommodate a range of travel of the actuator 549 corresponding to at least substantially the full range of travel of the plunger 57 in the cylinder 51 of the syringe 9. The coupling housing and coupling members described above may have other configurations.

The linkage 699 causes the plunger 57 of a syringe 9 to be released from the coupling 551 when the holding members 621 are moved to their release position. The linkage 699 comprises a pair of levers 701, one lever for each holding member 621 and associated coupling member 691. The levers 701 are positioned in the notches 623 in the bell-shaped holder housing 591 above respective holding members 621. Each of the levers 701 is mounted on a pin 751 bridging a respective notch 623 for pivoting about a generally horizontal axis generally parallel to the axis of the pivot pin 625 of the holding member 621 below. The levers 701 are lightly biased toward the position in Fig. 25 in which the lower end 753 of each lever is in contact with a surface on a respective holding member 621, and in which the upper end 755 of each lever is spaced from the a respective coupling member 691. The levers 701 are configured such they pivot into contact with the outer ends 733 of the coupling members 691 in response to pivotal movement of the holding members 621 to their release positions, e.g., when the release members 633 are pushed down to release a syringe 9. This contact moves the coupling members 691 to their de-coupling positions (Figs. 24 and 26). In this position, the lower faces 737 of the coupling members 691 are configured to assume a position (e.g., generally vertical) in which that they do not interfere with the exit of the plunger 57 from the coupling housing 671 as it falls from the chamber 681. Other linkage or release mechanisms may be used to release of the plunger from the coupling members.

Each of the exemplary syringe actuating devices 501, 503 described above has the ability to aspirate and dispense accurate quantities of materials having a high viscosity (e.g, up to at least about 2000 cps and preferably up to about 25,000 cps). This is due to the fact that a stepper motor 553 is used to closely control the movement of the actuator 549 while the cylinder 51 of the syringe 9 is securely held in position by the holder 545 and the actuator is securely coupled to the plunger 57 of the syringe. Thus, a large amount of force may be applied by the actuator 549 to the plunger to aspirate and dispense even very viscous materials (e.g., certain waxes, silicons, slurries containing such materials, etc.) in finely controlled quantities.

The lateral (Y-axis) spacing between the two actuating devices 501, 503 mounted on the robot 27 is desirably minimized to increase compactness of the syringe actuating apparatus 37 carried by the second arm 35 of the robot 27. To pick up a syringe 9, the robot 27 moves the second arm 35 to position one or the other of the syringe actuating devices 501, 503 to a position above a syringe in the rack 7. The respective cylinder 525 is then extended to move the bell-shaped holder housing 591 down to position in which the flange 65 on the cylinder 51 of the syringe 9 is gripped by respective holding members 621 (Fig. 25). The syringe 9 is then lifted from the rack 7 by the robot 27 (by moving the Z-member 407 on the second arm 35 of the robot vertically). The process can be repeated with the other of the actuating devices 501, 503 so that two syringes 9 are held concurrently by the syringe actuating apparatus 37. The syringes 9 are then transported to another part of the apparatus 1, such as the mixing station 11. Prior to a dispensing or aspirating step, the actuators 549 are coupled to the plungers 57 by operating the stepper motors 553 to move the actuators down until the plungers of the syringes 9 are received in the coupling housings 671 and gripped by the coupling members 691 (Fig. 25A). The use of two independently controlled syringes 9 carried by the same robot arm 35 in side-by-side relation provides increased throughput in carrying out different workflow procedures in that fewer trips between various stations of the apparatus 1 are required of the robot arm.

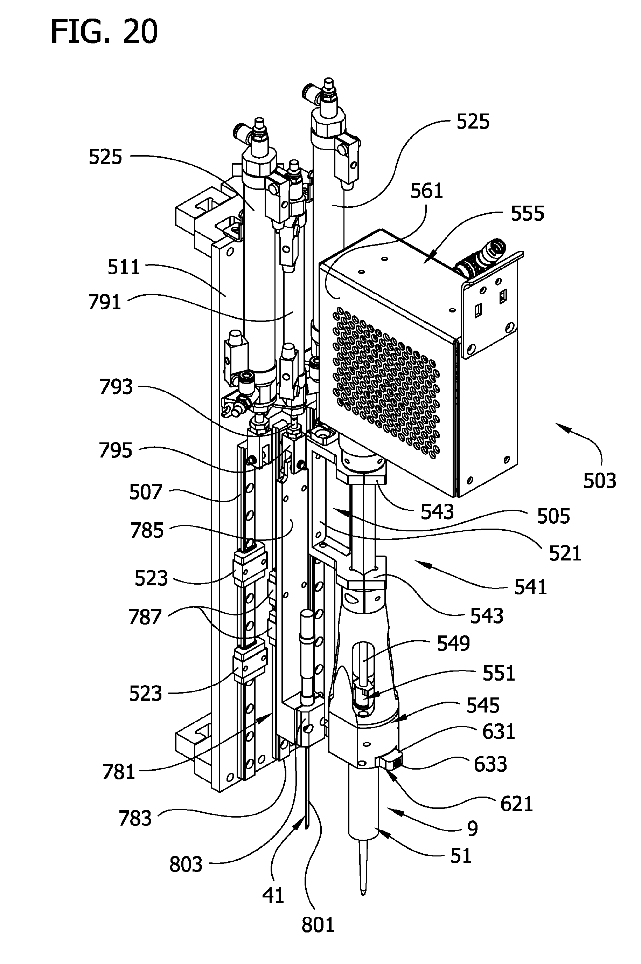

The sensing device 41 (Fig. 20) carried by the second arm 35 of the robot 27 is mounted on a separate carriage 781 which slides up and down on a vertical rail 783 on the mounting plate 511 secured to the vertically movable Z-member 407 on the second arm. In the embodiment of Fig. 20, the carriage 781 comprises a bracket 785 and a pair of vertically spaced sliders 787 fastened to the bracket 785 for sliding engagement with the rail 783. The carriage 781 is moved up and down on the rail 783 by a linear actuator 791, e.g., a pneumatic cylinder having a piston rod 793 with a clevis 795 connection to the carriage such that retraction of the rod causes the carriage to slide up on the rail and extension of the rod causes the carriages to slide down on the rail. The range of vertical movement of the sensing device 41 along the Z-axis is thus the combined range of vertical travel of the Z-member 407 on the second arm 35 and the vertical travel of the carriage 781 on the rail 783, the latter range corresponding to the stroke of the cylinder 791 moving the carriage. Desirably, the cylinder 791 is operable to move the sensor carriage 781 independent of the other carriages 505 carrying the syringe actuating devices 501, 503.

The sensing device 41 itself comprises a probe 801 secured to the carriage 781 by suitable means, e.g., a clamp member 803. The probe 801 senses one or more properties of the materials involved in the formulation process, such as pH. By way of example but not limitation, the probe 41 may have the following characteristics: maximum pH = 14; minimum pH = 0; maximum rate of pH measurement about 100 ms; and averaging of pH is possible.

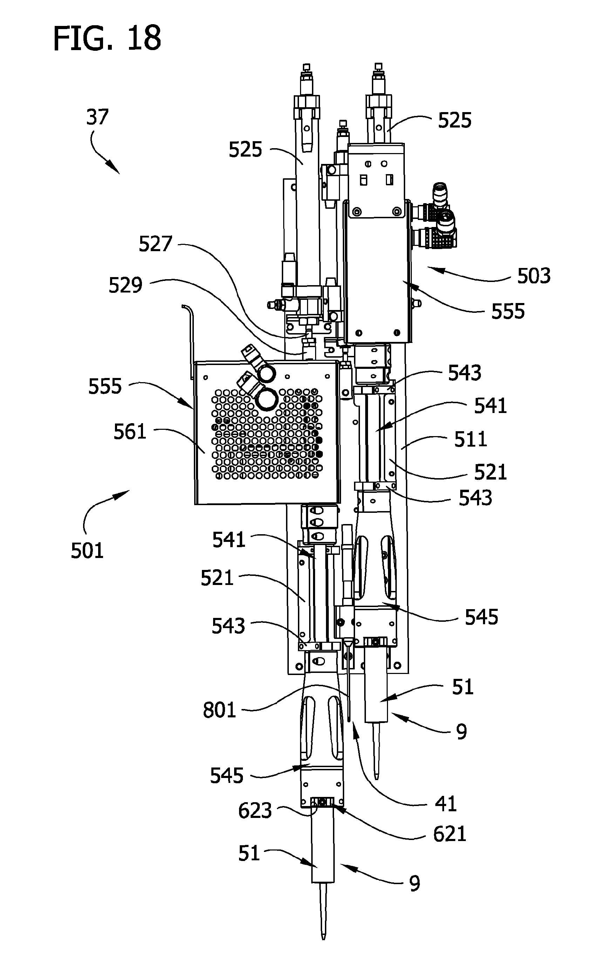

Referring to Figs. 18-20, the sensing device 41 is mounted on the second arm 35 of the robot 27 between the two syringe actuating devices 501, 503. Further, the motor housings 555 of the two syringe actuating devices 501, 503 are mounted at different orientations to provide a compact arrangement which allows one, two or all three devices 501, 503, 41 to be placed in a single container 15 at the same time. As a result, different materials can be dispensed into the container 15 in parallel (i.e., simultaneously or during overlapping time intervals) or in sequence, while also sensing at least one property of the materials in the container. Alternatively, the sensing device 41 may be used without any action by either or both syringe actuating devices 501, 503. The independent movement of the actuating and sensing devices 501, 503, 41 in the Z direction provides additional flexibility in maneuvering the devices during a procedure.
The devices 501, 503, 41 may be assembled and arranged in other ways.

The syringe disposal station 23 is equipped with a syringe release device 851 comprising a platform 853 supported by legs 855 on the deck 3 (Fig. 1). The platform 853 has a cantilever portion 857 projecting out over a side of the work station 1. A notch 859 in the cantilever portion 857 is sized and configured for accepting the housings 591 of the holders 545 of the syringe actuating devices 501, 503 such that the housing may be robotically moved into the notch 859 to a position in which the release members 633 are located directly below the platform 853. Actuation of the Z-axis rack 409 to raise the housing 591 causes the release members 633 to engage the platform 583, thereby pivoting the holding members 621 to their release positions and the coupling members 691 to their de-coupling positions to allow the syringe 9 to drop down and out of the syringe actuating apparatus 37 into a suitable waste receptacle (not shown) for disposal.

The work station 1 also includes a conventional wash station 45 for washing and drying the sensing probe 41 and syringe nozzles 59. A number of source vials 47 containing selected materials (e.g., pH solutions) are located adjacent the washing and drying station 45.

The operation of the robot 27, the syringe actuating devices 501, 503, the sensing device 41, weighing apparatus 21, and the temperature control systems 121 are under the control of suitable controller (not shown) comprising a processor and associate hardware and software. The software may include Renaissance Impressionist® and Epoch® software, available from Symyx Technologies, Inc. (Santa Clara, California).

The work station 1 described above can be used to conduct a variety of work flows or processes, e.g., the preparation of catalyst slurries and pharmaceutical mixtures, particularly those involving viscous materials. For example, in one procedure to prepare catalyst slurries, catalyst materials are processed by combining starting materials (sources) using either a regular addition process (moving a slurry from one container to another), pH control addition (adding an acid and base solution to a container simultaneously while maintaining a constant pH), or pH adjustment (adding either an acid or a base to change the pH of the contents of a container to a new value).

In a regular addition process, the robot 27 is programmed to pick up two syringes 9 from the syringe rack 7 and to transport them to a source container 15 at the mixing station 11 to aspirate predetermined volumes of material into the syringes. Prior to aspiration, any mixing operation which may be taking place in the source container 15 is stopped, and the mixing blade(s) 173 in the container come to rest at a known ("home") rotary position. Taking this position into account, a syringe 9 carried by the robot 27 is moved to a position which provides an unobstructed path down past the blade(s) 173 to the bottom wall 137 of the container 15. The cylinder 525 is then extended to move the syringe 9 down to a position in which its nozzle tip 62 is immediately adjacent the bottom wall 137 of the container 15. In this position, the stepper motor 553 is activated to move the elongate actuator 549 to retract the plunger 57 in its respective cylinder 51 to aspirate a predetermined amount of source material into the syringe 9. Upon completion of the aspiration step the pneumatic cylinder 525 is actuated to raise the syringe 9 for transport by the robot 27 to positions above a target container 15, such as an "intermediate" container containing a mixture of one or more materials. The stepper motor 553 is then actuated to extend the plunger 57 in their respective cylinder 51 to dispense a predetermined amount of source material from the syringe 9 into the target container 15.

The two syringes 9 may be used in a variety of ways to perform a variety of different workflows. By way of example but not limitation, one syringe 9 may be used to aspirate a first source material and the other syringe may be used to aspirate a second source material. These materials may then be dispensed into the same target container 15 or different target containers. Similarly, after materials have been mixed in the intermediate containers 15, the same or different syringes 9 can be used to transfer materials from the intermediate containers to one or more destination containers in any desired sequence. Because the syringe actuating devices 501, 503 operate the syringes 9 independently of one another, two syringes can be operated in parallel (i.e., in the same or overlapping time periods) or in sequence to aspirate materials from one or more of the containers 15 and dispense materials into one or more containers in a single trip of the second robot arm 35. For example, both syringes 9 can be lowered into the same container 15 (in parallel or in sequence) to aspirate a double volume of material from that container for dispensing into one or more other containers. The extension and retraction of the plungers 57 for aspiration may be carried out in parallel (i.e., in the same or overlapping time periods) or in sequence, depending on which is more desirable for a particular workflow. If the amount of material to be dispensed into the target container 15 exceeds the combined volumes of the syringes 9, the aspiration/dispense process is repeated until the required amount has been added to the one or more target containers. The contents of the containers 15 are mixed as needed by energizing the appropriate mixing motors 179.

In a pH control addition procedure, the sensing device 41 comprises a pH probe that is suitably calibrated using a number of calibrating materials in selected containers 15 and then washed at the washing station 45. Using the syringe actuating devices 501, 503, the robot 27 picks up two syringes 9 from the rack 7, one after the other, and aspirates an acid material from a first source container 15 into one syringe and a base material from a second source container into the other syringe using the techniques previously described. Upon completion of the aspiration, the robot 27 moves the syringes 9 to a target container 15. If that container contains material, the sensing device cylinder 791 is actuated to move the pH probe 41 into the container 15 to take a measurement. After a measurement is taken, the syringes 9 are actuated to dispense both acid and base materials into the container 15. The base material is used to control the pH of the mixture while the acid material is added at a constant rate. The pH of the materials in the container is recorded by the sensing device 41 at periodic intervals (e.g., about every two seconds) and the dispensing rate of the base material is varied by the respective stepper motor 553 to maintain a constant pH of the mixture. After the appropriate amount of base and acid materials have been robotically dispensed, the cylinders 525, 791 are actuated to retract the actuating and sensing devices 501, 503, 41, and the robot 27 transports the syringes 9 to the disposal station 23 for disposal in the manner described above.

In a pH adjustment procedure, the robot 27 picks up a syringe 9 from the syringe rack 7 and aspirates a predetermined amount of acid or base material from a source container 15, using the techniques previously described. The acid or base material is dispensed into a target container 15 containing materials. The acid or base material is dispensed at a constant rate as the contents of the container are mixed. The sensing device 41 is used to measure the pH of the contents of the container. When the pH of the contents of the container reaches a predetermined value, dispensing is stopped and the syringe 9 is transported to the disposal station 23 for disposal.

One or more of the exemplary procedures described above may be incorporated into a workflow which also includes various mixing and/or weighing steps. During a mixing step, the mixing blades 173 in one or more containers are rotated at the appropriate speeds to effect suitable mixing of the contents of the one or more containers 15. Further, at selected times, a container 15 may be robotically removed from its respective well 103 (using the gripping mechanism 33) and transported to the weighing station 19 where the container is placed on the pedestal 311 of weigh cell 301 for weighing. After the container 15 and its contents have been weighed, the container is robotically transported back to its well 103 and lowered to re-couple the mixing shaft 185 to the drive shaft 187. The ability to robotically weigh a container 15 and its contents at any time during a procedure is advantageous for determining such properties as the density of the materials being prepared.

The temperature of the materials in the containers 15 can also be closely controlled and monitored during a workflow by using the temperature control system 121 previously described.

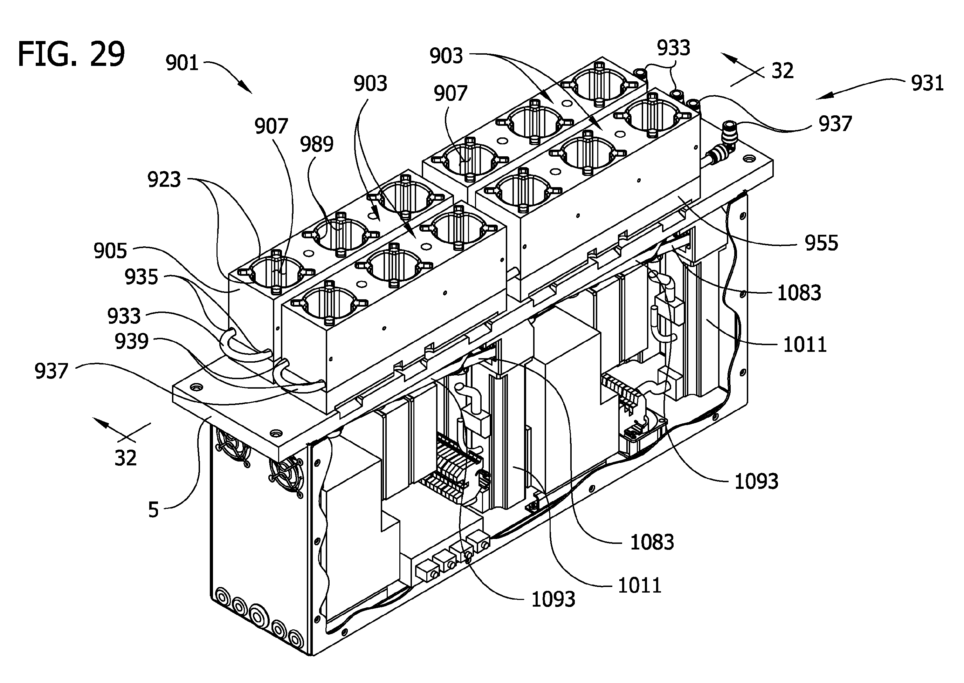

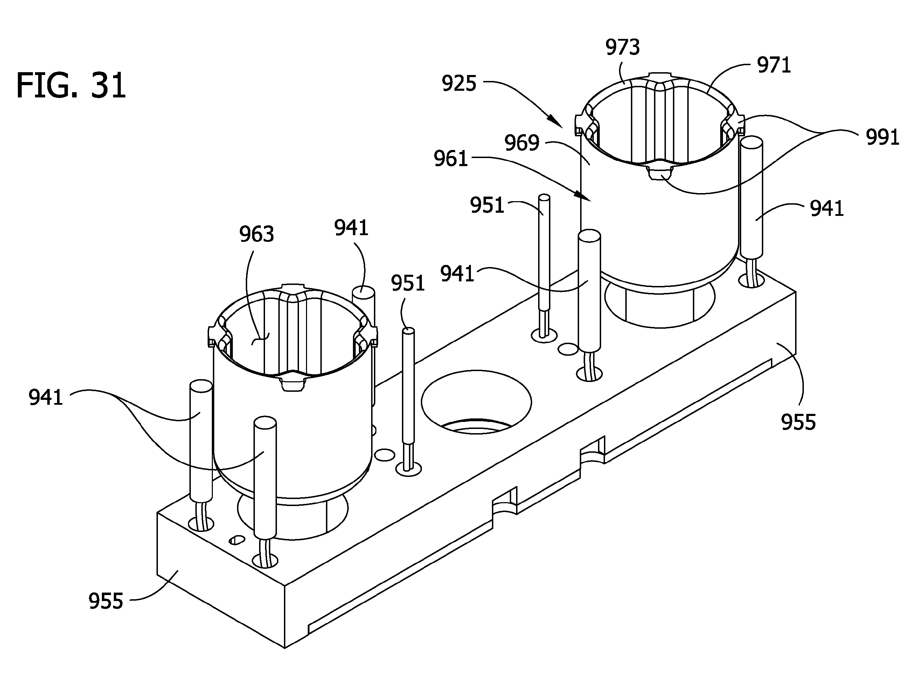

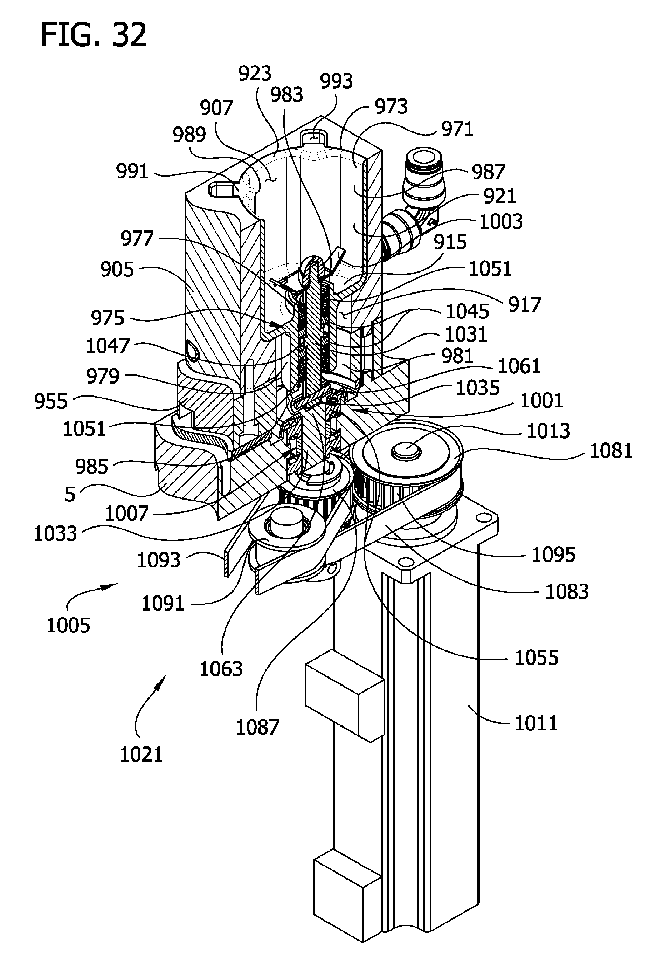

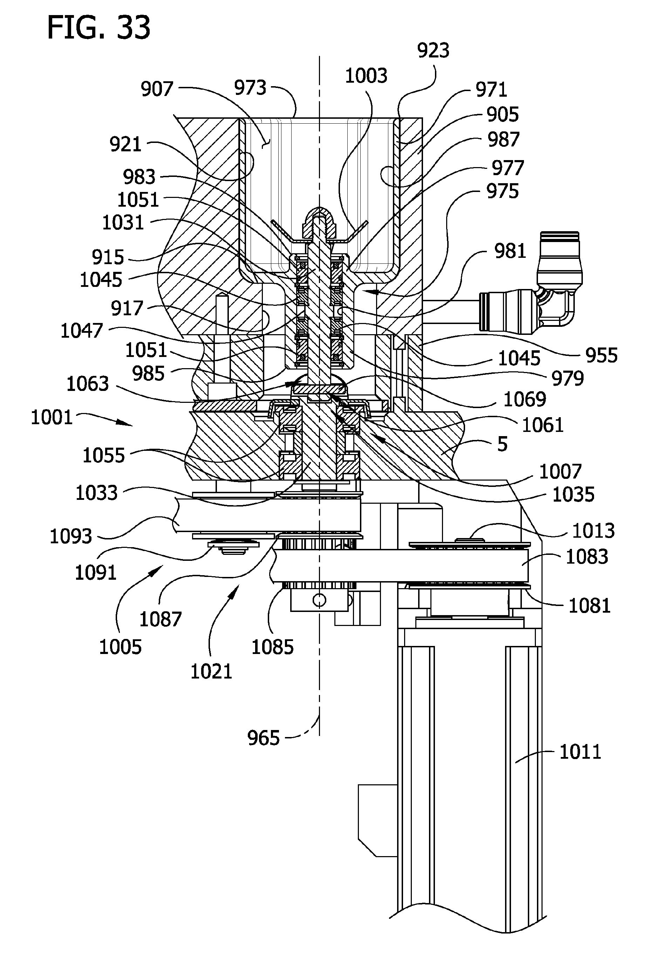

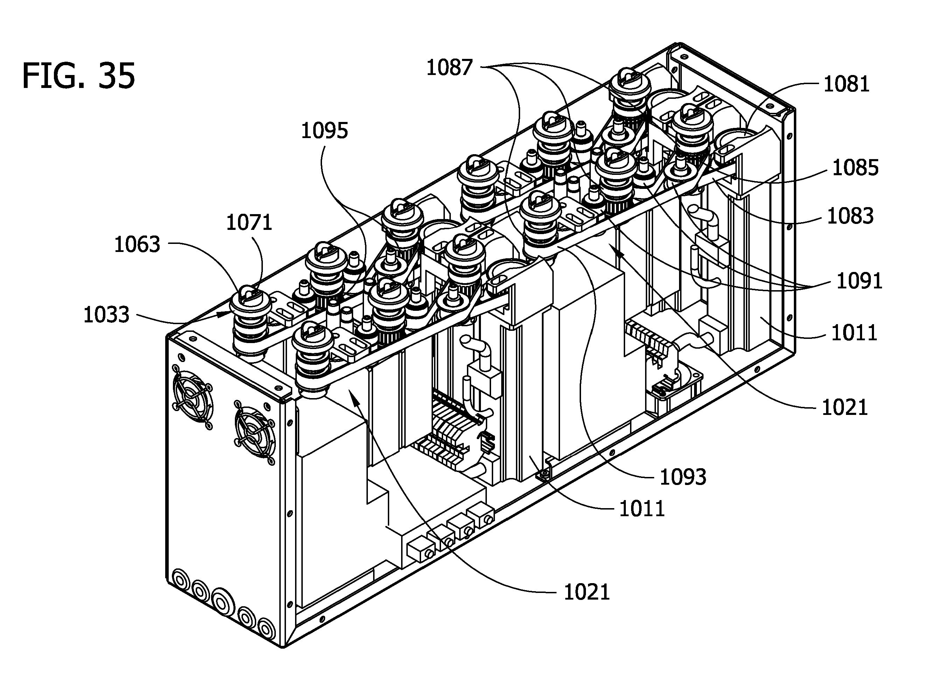

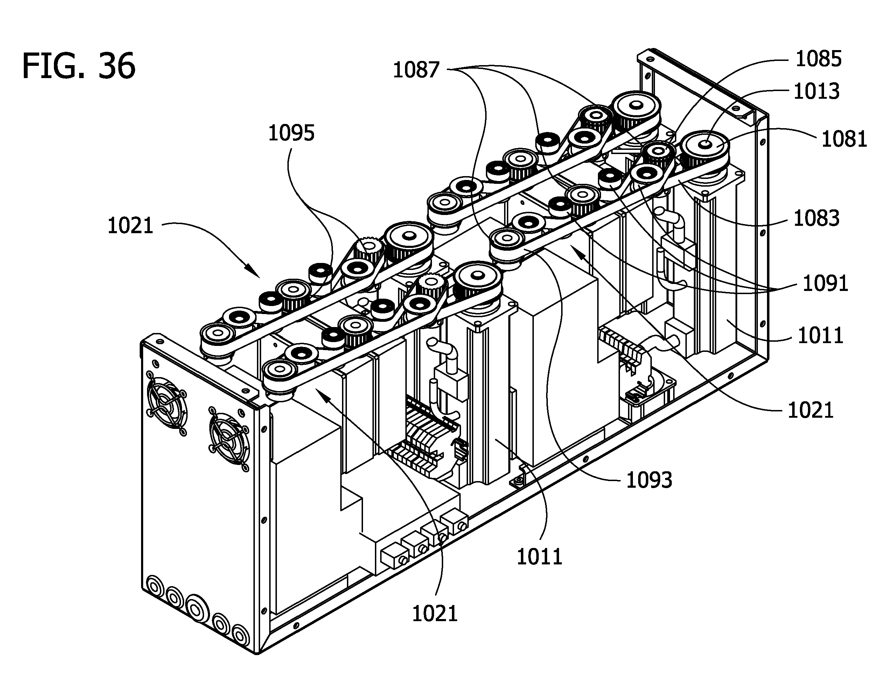

Figs. 29-36 illustrate a second embodiment of apparatus to be used at the mixing station for making various types of formulations, e.g., paints, make-up emulsions, and other products. The apparatus, generally designated 901, comprises a plurality of mixing modules 903 for holding arrays of containers 925. In this particular embodiment, four such modules 903 are illustrated, each comprising a metal mixing block 905 configured with three openings or wells 907 for a row of three containers 925, but this number and configuration of wells can vary. Each well 907 has a bottom surface 915 (Figs. 32 and 33), a central bore 917 extending down through the bottom surface, a side surface 921 extending up from the bottom surface, and an open top 923. The well 907 is sized and configured for holding one container 925. Preferably, a temperature control system 931 is provided for controlling the temperature of the materials in the containers 925. As illustrated in Fig. 29, the temperature control system 931 comprises a first conduit 933 extending through bores 935 in two of the four blocks 905 for the flow of heating or cooling fluid through the blocks and a second conduit 937 extending through bores 939 in the other two blocks for the flow of heating or cooling fluid through the blocks. Desirably, as shown in Fig. 32, one or more independently controlled heaters 941, e.g., electrical resistance cartridge heaters, are provided in bores (not shown) in the blocks 905 adjacent each well 907. The temperatures of the mixing blocks 905 are monitored using suitable temperature sensing devices 951, e.g., thermocouples. This arrangement allows the temperature of each mixing block 905 to be controlled independent of other blocks. The mixing blocks 905 rest on insulating pads 955 which function to thermally isolate the blocks from the deck 3. The blocks 905 and pads 955 are secured to the deck panel 5 by suitable fasteners.

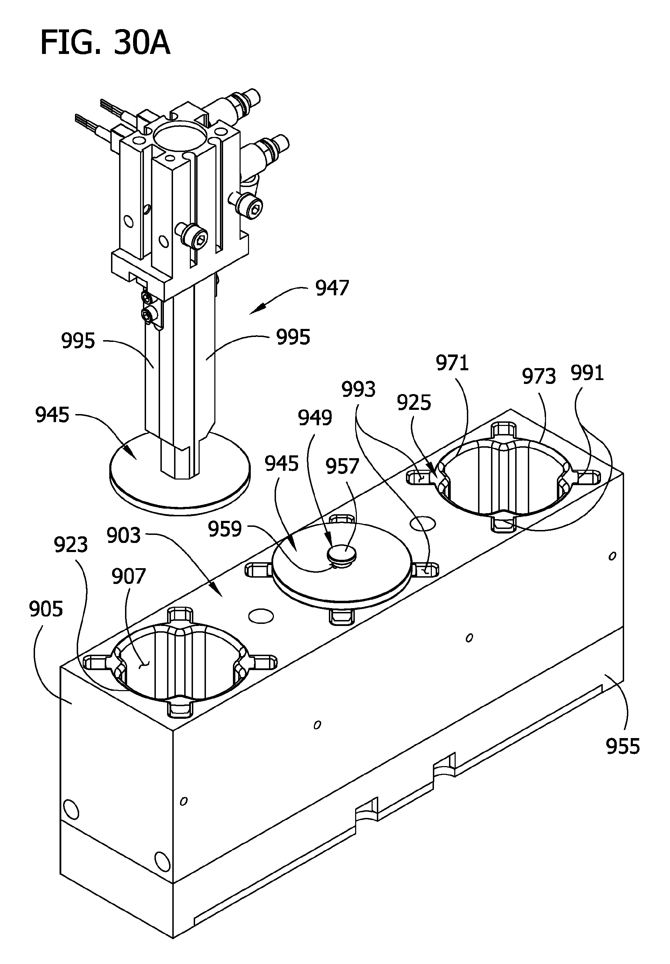

Referring again to Figs. 32 and 33, each container 925 comprises a cup-shaped body 961 defining a volume 963 for holding material, a generally vertical central axis 965, a bottom wall 967, an upstanding generally cylindrical side wall 969, and an upper rim 971 defining an open upper end 973 of the container. The bottom wall 967 of the container 925 includes a central hub 975 having an upper portion 977 which extends up into the interior volume 963 of the container and a lower portion 967 which projects down into the bore 917 in the bottom surface 915 of the well 917 when the container is seated in its respective well. A bore 981 co-axial with the central axis 965 of the body 961 extends through the hub 975 from its upper end 983 to its lower end 985. The inner surface 987 of the container side wall 969 comprises one or more vertical ribs 989 which project inward toward the central axis 965 of the container 925. A number of lugs 991 or keys project outward from the rim 971 of the container 925 for receipt in recesses 993 in the top surface of a respective block 905 to maintain the containers in proper registration (i.e., angular orientation) with respect to their respective wells 907 (see Figs. 30A-D and 32). For reasons that will become apparent, the recesses 993 are longer than the lugs 991 and extend deeper into the block 905 than the lugs.

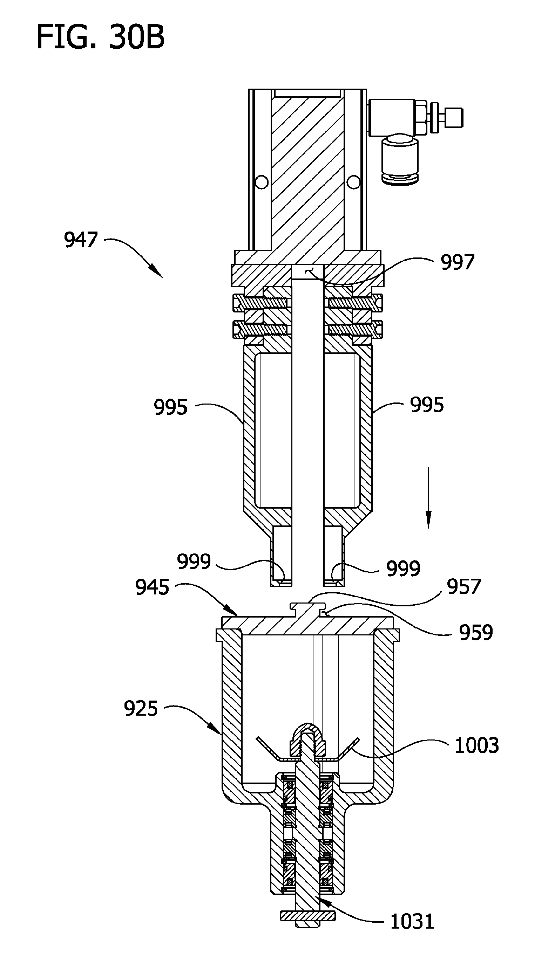

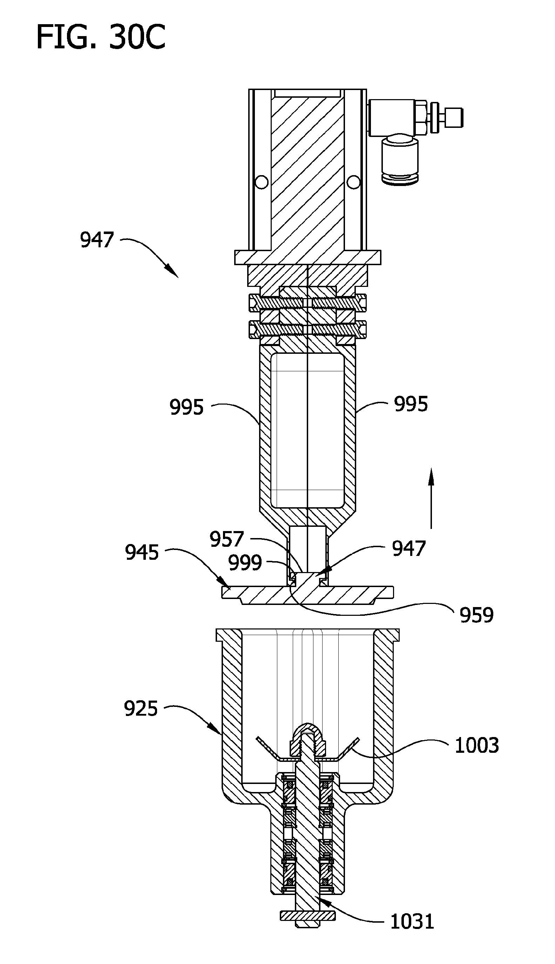

As shown in Figs. 30A-C, caps 945 can be positioned to cover the open tops 973 of one or more containers 925 to limit evaporative losses of materials in the containers if desired. The caps 945 shown in the drawings are held on the containers 925 by gravity and can be lifted from the containers without pulling the containers out of mixing module block 905. The first arm 31 of the robot 27 is optionally equipped with a cap lifting apparatus 947 to enable the robot to lift the caps 945 off the containers 925 to access the materials in the containers (e.g., for testing with the probe 801), add materials to the containers, and/or remove materials from the containers. In the embodiment illustrated in Figs. 30A-30C, each of the caps 945 has a handle 949 (e.g., a knob 957 defining a circumferential groove 959) to facilitate lifting the respective cap off its container 925. The cap lifting apparatus 947 suitably comprises two clamp members 995 moveable, e.g., by a pneumatic cylinder (not shown), in a slot 997 between an open position (Fig. 30B) and a closed position (Fig. 30C). Arcuate flanges 999 extend radially inward from the lower ends of the clamp members 995. The flanges 999 are sized and shaped so that they may be received in the groove 959 to connect the clamp members 995 to the handle 949 so that the clamp members can lift the cap 945 from the container 925.

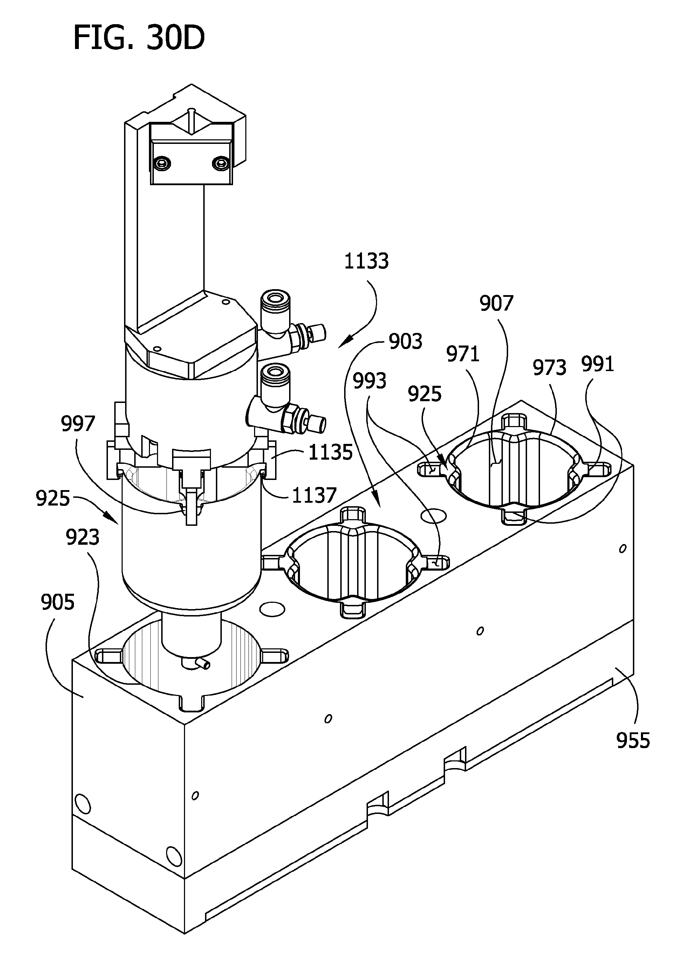

Another embodiment of a container gripper mechanism 1133 is carried by the first arm 31 of the robot 27 in lieu of the gripping mechanism 33 described above. The gripping mechanism 1133 is suitable for use with the containers 925 of this embodiment. The gripping mechanism comprises a plurality of grippers 1135 (e.g., four grippers are shown in Fig. 30D) having inward projecting fingers 1137 sized and configured for lifting a container 925 by its lugs 991. The grippers 1135 are operated by suitable means (e.g., pneumatics) to move between a non-gripping or release position in which the grippers are extended radially outward for lowering the fingers 1137 into the recesses 993 outward of the lugs 991, and a gripping position in which the grippers are contracted radially inward so that the fingers move beneath the lugs in the recesses so that the lugs are supported by the fingers when the robot 27 raises the gripping mechanism.

The mixing modules 903 are equipped with apparatus 1001 for mixing the contents of the containers 925. In particular, this apparatus 1001 includes mixing blades 1003 in the containers 925 and a drive system 1005 associated with each 1x3 array of containers for rotating the blades in the containers. The drive system 1005 comprises a number of direct drive mechanisms 1007, one for each container 925, extending up through the bottom walls 967 of the containers for rotating the mixing blades 1003, a motor 1011 secured to the underside of the deck panel 5 and having an output shaft 1013, and a belt-and-pulley system 1021 coupling the motor to the direct drive mechanisms for rotating the mixing blades.

As shown in Figs. 32 and 33, each direct drive mechanism 1007 includes a mixing shaft 1031 extending up through the hub 975 in the container, a drive shaft 1033 for rotating the mixing shaft, and a releasable coupling 1035 for releasably connecting the drive shaft and the mixing shaft. The upper end of the mixing shaft 1031 projects above the hub 975 into the interior volume 963 of the container 925. The mixing blade or blades 1003 are secured to the mixing shaft 1031 adjacent the upper end of the shaft by one or more fasteners (not shown) for convenient removal and replacement of the blade(s). The configuration of the blade(s) will vary, depending on the materials being mixing, the mixing speed and other factors known to those skilled in this art. Preferably, the mixing blades 1003 used in each 1x3 array of containers 925 are of identical configuration.

The mixing shaft 1031 of each direct drive mechanism 1007 rotates in upper and lower bearings 1045 (e.g., ball bearings) mounted in the bore 981 of the hub 975 of a respective container 925 (Figs. 32 and 33). The bearings 1045 are maintained spaced apart by radial flange 1047 on the mixing shaft 1031. Upper and lower seals 1051 in the bore 981 above and below the bearings 1045 seal the bearings against contamination from materials inside or outside the container 925. This sealing arrangement allows the entire unit (container 925, mixing blade(s) 1003 and mixing shaft 1031) to be placed in a bath for cleaning.

The drive shaft 1033 of each direct drive mechanism 1007 rotates in a pair of bearings 1055 mounted in an opening 1057 in the deck panel 5 of the workstation. The drive shaft 1033 extends down below the deck panel 5 for driving engagement by the belt-and-pulley system 1021.

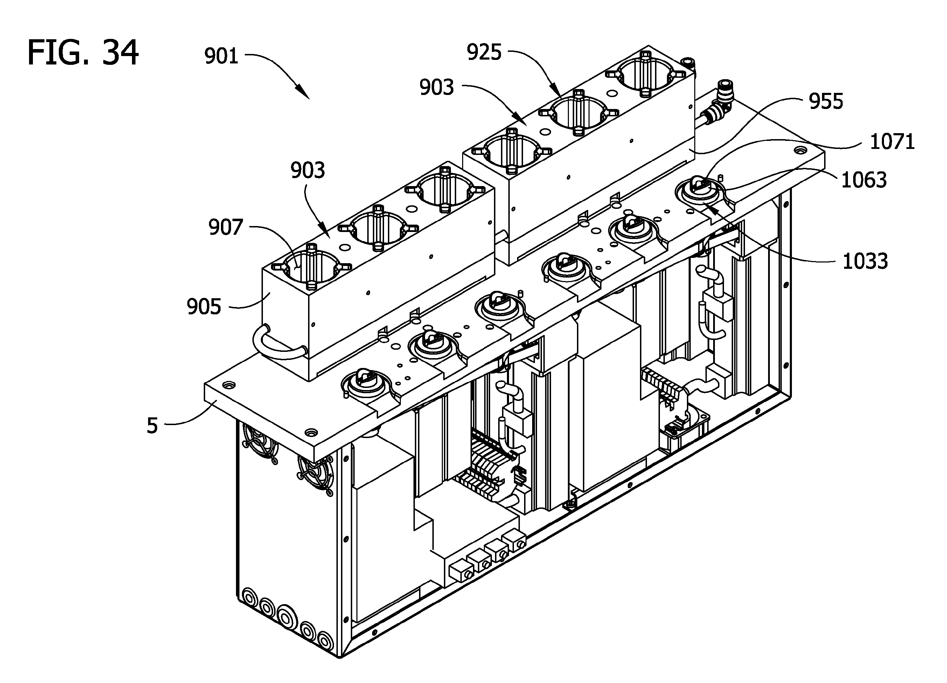

The coupling 1035 between each drive shaft and mixing shaft is illustrated in Figs. 33 and 34. The coupling 1035 comprises an upper coupling member 1061 on the lower end of the mixing shaft 1031, and a lower coupling member 1063 on the upper end of the drive shaft 1033. In this particular embodiment, the upper coupling member 1061 comprises a horizontal pin 1069 projecting laterally from the mixing shaft 1031 and the lower coupling member 1063 comprises a slot 1071 in the upper end of the drive shaft 1033 for receiving the pin on the mixing shaft. Relative rotation between the two shafts 1031 is substantially prevented 1033 when the pin 1069 is received in the slot 1071, as during a mixing operation. The coupling 1035 allows axial (e.g., vertical) separation of the two shafts 1031, 1035 so that the container 925, mixing shaft 1031, upper coupling member 1061, and mixing blade(s) 1003 can be removed as a unit from the well 907. Thereafter, the container 925 can be replaced in the well 907 to re-couple the mixing and drive shafts 1031, 1035 for another mixing operation. Other releasable couplings between the mixing and drive shafts 1031, 1035 can be used without departing from the scope of this invention.

Figs. 34-36 illustrate the belt-and-pulley systems 1021 for rotating the various drive shafts 1033 associated with the mixing modules 903. The three drive shafts 1033 of each module 903 are driven by a common motor 1011 having an output shaft 1013 which mounts a drive pulley 1081 connected by a first belt 1083 to a first or "master" gear 1085 on one of the drive shafts 1033 of the module 903 (Fig. 33). Each system 1021 also includes a slave pulley 1087 mounted on the lower end of each of the remaining drive shafts 1033 (i.e., the other two drive shafts in the exemplary 3-container mixing module), a plurality of idler rollers 1091, and a second belt 1093 trained around the master gear 1085, slave pulley(s) and idler rollers. Thus, rotation of the motor output shaft 1013 rotates all of the drive shafts 1033, mixing shafts 1031 and mixing blades 1003 in the same direction to effect mixing of the materials in the containers 925. Desirably, the first and second belts 1083, 1093 are timing belts having teeth (not shown) which engage complementary teeth 1095 on the pulleys 1081, 1087 so that rotation of the drive and mixing shafts 1031, 1033 can be maintained in timed relation and angular orientation relative to one another. The master gear 1085 and slave pulleys 1087 are secured in fixed position to respective drive shafts 1033 by split clamps (not shown) or other means which can be loosened to allow adjustment of the relative angular positions of the shafts, and then re-tightened. Other belt and pulley systems and other types of drive systems may be used without departing from the scope of the invention.

The motor 1011 is controlled by a suitable controller (not shown) to rotate the direct drive mechanisms 1007 and mixing blades 1003 at the same selected mixing speed and in synchronization such that the mixing blades all have the same angular (rotary) position at any given time. Further, at the end of a mixing interval, the controller is programmed to operate the motor 1011 to move all of the direct drive 1007 mechanisms of a respective mixing module 903 to a predetermined "home" position in which the mixing blades 1003 in the containers 925 reside in a known rotary position to provide an unobstructed path extending from the open top 973 of the container down past the mixing blades to the bottom wall 967 of the container. In this manner, the robot 27 can be operated to move a syringe 9 down along an unobstructed path to an aspirating position immediately adjacent the bottom wall 967 of the container 925, taking into account the predetermined rotary position of the mixing blade(s) 1003 in a container so that there is no contact between the blade(s) and the syringe.

Using the mixing station 901 in combination with the workstation 1 provides the flexibility to develop workflows for preparing and testing various formulated materials, including materials in which the components and/or the material as formulated are very viscous. For instance, one exemplary workflow is suitable for preparing and testing various high viscosity formulated personal care products (e.g., lotions, shampoos, cosmetics, skin creams, and the like).

If caps 945 are used, they are suitably on the containers 925 at the start of the workflow. Any time a cap 945 needs to be removed from a container 925 (e.g., to add materials, remove materials, test materials with the probe 801, etc.) the cap can be lifted by the cap lifting apparatus 947. To lift a cap 945 from a container 925, the robot 27 positions the clamp members 995 in alignment with the handle 947 and lowers the clamp members 995 in their open position until the flanges 999 are even with the groove 959. Then clamp members 995 are moved to their closed position so that the flanges 999 are received in the groove 959. With the clamp members 995 in their closed position, the knob 957 is supported by the flanges 999 so that the cap 945 is lifted off of the container 925 by upward movement of the clamp members in their closed position by the robot 27.

Formulated test materials are suitably prepared in the containers 925 by using the syringe actuating apparatus 37 in the same manner described above to transfer metered quantities of ingredients (e.g., solvents, surfactants, waxes, emulsifiers, fragrances, silicons, etc.) of the formulated product into the containers 925. Any time mixing is desired, the mixing apparatus 1001 is activated to mix the materials using the mixing blades 1003. The weigh station 19 can be used to weigh a container 925 whenever desired. For example, the robot 27 can pick up a container 925 using the container gripping mechanism 1133 and move it to the weigh cell 301 before and after addition of any ingredients that are key to the particular experiment being conducted (or each of the ingredients if desired) to verify that the intended amount of that ingredient has been added to the container 925 by the syringe actuating apparatus 37.

The temperature control system 931 provides various options for controllably adjusting the temperature of the materials in the containers 925 (e.g., to produce a phase change of one or more materials in the containers). For example, in one embodiment of a workflow, heat from the heaters 941 is used to melt the materials in the containers 925 or maintain molten materials in a molten state while the mixing blades 1003 stir the materials in the container. Then the materials are allowed to cool in the containers 925 (e.g., by turning off the heaters 941 and/or pumping a fluid through the conduits 933, 937) in order to study crystallization properties resulting from the cooling of the formulated material.

Whenever desired, the probe 801 can be lowered into any of the containers 925 to measure properties of the materials therein. In the workflow described above, for example, the probe 801 may be equipped with a conductivity sensor (not shown) to measure conductivity of the formulated personal care product (e.g., before and/or after crystallization of the product) in the container 925.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the invention are achieved and other advantageous results attained.

As various changes could be made in the above constructions and methods without departing from the scope of the invention, it is intended that all matter contained in the above description and shown in the accompanying drawings shall be interpreted as illustrative and not in a limiting sense. It follows a list of further embodiments of the invention
Embodiment 1. Apparatus for actuating one or more syringes, each syringe comprising a cylinder having upper and lower ends, and a plunger movable in the cylinder, said apparatus comprising
   a programmable robot, and
   at least one syringe actuating device mounted on the robot for actuating a syringe to transfer material to or from a container;
   said at least one syringe actuating device comprising
      a holder for holding and releasing the cylinder of a syringe,
      an actuator movable relative to the holder, and
      a coupling for coupling the actuator to the plunger of the syringe such that, when the cylinder of the syringe is held by the holder, movement of the actuator in a first direction causes the plunger to move up in the cylinder and movement of the actuator in a second direction causes the plunger to move down in the cylinder.
Embodiment 2. Apparatus with the features of embodiment 1 wherein said robot comprises an arm movable in a generally horizontal plane along X and Y axes, and a Z-member mounted on the arm for generally vertical movement along a Z axis, said syringe actuating device being mounted on the Z-member of the robot.
Embodiment 3. Apparatus with the features of embodiment 2 wherein said at least one syringe actuating device is movable relative to said Z-member in a generally Z-direction.
Embodiment 4. Apparatus with the features of embodiment 3 wherein said at least one syringe actuating device is mounted on a carriage movable on a rail secured in fixed position relative to said Z-member.
Embodiment 5. Apparatus with the features of embodiment 2 further comprising a sensing device mounted on the Z-member of the robot adjacent said at least one syringe actuating device.
Embodiment 6. Apparatus with the features of embodiment 5 wherein said sensing device is movable relative to the Z-member in a generally Z-direction.
Embodiment 7. Apparatus with the features of embodiment 1 further comprising a sensing device mounted on the robot adjacent said at least one syringe actuating device, said sensing device being operable to sense a property of material in said container.
Embodiment 8. Apparatus with the features of embodiment 7 comprising at least two of said syringe actuating devices mounted on the robot adjacent said sensing device.
Embodiment 9. Apparatus with the features of embodiment 1 comprising at least two of said syringe actuating devices mounted adjacent one another on the robot.
Embodiment 10. Apparatus with the features of embodiment 1 wherein said holder comprises a housing having an open lower end to permit a syringe to be entered into the housing to a position to be held by the holder and to exit the housing upon release by the holder, and at least one holding member mounted on the housing for movement between holding and release positions.
Embodiment 11. Apparatus with the features of embodiment 10 wherein said at least one holding member is pivoted on the housing for movement between said holding position in which the holding member is positioned to support the cylinder in fixed position relative to the housing and said release position in which the holding member does not support the cylinder.
Embodiment 12. Apparatus with the features of embodiment 10 wherein said at least one holding member is configured for engagement with a flange on the cylinder of the syringe when the holding member is in said holding position
Embodiment 13. Apparatus with the features of embodiment 3 further comprising a release mechanism on the housing adapted to be actuated for moving the at least one holding member to said release position.
Embodiment 14. Apparatus with the features of embodiment 13 further comprising a deck, a rack on the deck for supporting a plurality of syringes in position for loading into the housing of the holder, and a syringe release device on the deck configured to interact with said release mechanism to release a syringe from the housing for disposal.
Embodiment 15. Apparatus with the features of embodiment 1 wherein said coupling is a releasable coupling movable between a coupling position for coupling the actuator to the plunger of the syringe and an de-coupling position for de-coupling the actuator from the syringe.
Embodiment 16. Apparatus with the features of embodiment 15 wherein the coupling is movable to its de-coupling position in response to movement of the at least one holding member toward said release position.
Embodiment 17. Apparatus with the features of embodiment 16 further comprising linkage operable in response to movement of the at least one holder member toward said release position to move the coupling to its de-coupling position.
Embodiment 18. Apparatus with the features of embodiment 1 wherein said at least one actuating device comprises a stepper motor for moving said actuator.
Embodiment 19. Apparatus adapted for use with a programmable robot to actuate one or more syringes, each syringe comprising a cylinder having upper and lower ends and a plunger movable up and down in the cylinder, said apparatus comprising
   at least one syringe actuating device adapted to be mounted on a robot, said device comprising
      a holder for holding the cylinder of the syringe, said holder comprising at least one holding member movable between a holding position for holding the cylinder and a release position for releasing the cylinder, and
      an actuator for moving the plunger of the syringe in the cylinder while the cylinder is held by the holder, and
      a coupling for coupling the actuator to the plunger of the syringe such that upward movement of the actuator causes the plunger to move up in the cylinder and downward movement of the actuator causes the plunger to move down in the cylinder.
Embodiment 20. Apparatus with the features of embodiment 19 further comprising a sensing device assembled with the at least one syringe actuating device for sensing a property of material dispensed from or aspirated by a syringe actuated by the syringe actuating device.
Embodiment 21. Apparatus with the features of embodiment 20 comprising two of said syringe actuating devices assembled to reside side-by-side.
Embodiment 22. Apparatus with the features of embodiment 19 comprising two of said syringe actuating devices assembled to reside side-by-side.
Embodiment 23. Apparatus with the features of embodiment 19 wherein said holder comprises a housing having an open lower end to permit a syringe to be entered into the housing to a position to be held by the holder and to exit the housing upon release by the holder, and at least one holding member mounted on the housing for movement between holding and release positions.
Embodiment 24. Apparatus with the features of embodiment 23 wherein said at least one holding member is pivoted on the housing for movement between said holding position in which the holding member is positioned to support the cylinder in fixed position relative to the housing and said release position in which the holding member does not support the cylinder.
Embodiment 25. Apparatus with the features of embodiment 23 wherein said at least one holding member is configured for engagement with a flange on the cylinder of the syringe when the holding member is in said holding position.
Embodiment 26. Apparatus with the features of embodiment 23 further comprising a release mechanism on the housing adapted to be actuated for moving the at least one holding member to said release position.
Embodiment 27. Apparatus with the features of embodiment 26 further comprising a deck, a rack on the deck for supporting a plurality of syringes in position for loading into the housing of the holder, and a syringe release device on the deck configured to interact with said release mechanism to release a syringe from the housing for disposal.
Embodiment 28. Apparatus with the features of embodiment 19 wherein said coupling is a releasable coupling movable between a coupling position for coupling the actuator to the plunger of the syringe and an de-coupling position for de-coupling the actuator from the syringe.
Embodiment 29. Apparatus with the features of embodiment 28 wherein the coupling is movable to its de-coupling position in response to movement of the at least one holding member toward said release position.
Embodiment 30. Apparatus with the features of embodiment 29 further comprising linkage operable in response to movement of the at least one holder member toward said release position to move the coupling to its de-coupling position.
Embodiment 31. Apparatus with the features of embodiment 19 wherein said at least one actuating device comprises a stepper motor for moving said actuator.
Embodiment 32. A method of robotically dispensing material from a syringe, comprising
   robotically moving at least one syringe to a position for dispensing into a container,
   robotically moving a plunger of the at least one syringe to dispense material from the syringe into the container, and
   robotically sensing a property of the material dispensed into the container.
Embodiment 33. A method with the features of embodiment 32 further comprising simultaneously robotically moving two syringes to a position above the container, dispensing different materials from the syringes into the container, and mixing the materials in the container at a mixing station.
Embodiment 34. A method with the features of embodiment 33 wherein said sensing step is effected in parallel with said step of dispensing into the container.
Embodiment 35. A method with the features of embodiment 34 wherein different materials are dispensed by the two syringes and the amount of material dispensed from one or both of the syringes is varied in response to the sensed property.
Embodiment 36. A method with the features of embodiment 35 wherein the sensed property is pH and the amount of material dispensed by one or both syringes is varied to control the pH of the materials as they are mixed.
Embodiment 37. A method with the features of embodiment 32 further comprising robotically transporting the container containing mixed materials from the mixing station to a weighing station, and weighing the contents of the container.
Embodiment 38. A method with the features of embodiment 37 further comprising robotically transporting the container from the weighing station back to the mixing station, dispensing additional material from one or both of said syringes into the container, and mixing the materials.
Embodiment 39. A method of robotically dispensing from two or more syringes, comprising
   robotically moving two or more syringes to a location for dispensing or aspirating materials, and
   robotically moving plungers of the two or more syringes in parallel for dispensing or aspirating material.
Embodiment 40. A method with the features of embodiment 39 further comprising moving said plungers independent of one another.
Embodiment 41. A method with the features of embodiment 39 wherein said plungers are robotically moved to dispense materials into the same container.
Embodiment 42. A method with the features of embodiment 39 wherein further comprising sensing a property of material in a container into which said material is dispensed, and varying the rate of dispensing from one of the syringes in response to the sensing.
Embodiment 43. A method with the features of embodiment 42 wherein said sensed property is pH.
Embodiment 44. A method of robotically actuating disposable syringes, each syringe comprising a cylinder and a plunger movable in the cylinder, said method comprising the steps of
   a) placing a plurality of disposable syringes in a rack,
   b) robotically removing a selected syringe from the rack and transporting the syringe to a material transfer station,
   c) robotically moving the plunger of the syringe in the cylinder to transfer material to or from the syringe, and
   d) robotically disposing of the syringe.
Embodiment 45. A method with the features of embodiment 44 wherein step (d) comprises robotically transporting the syringe to a syringe disposal station, and robotically releasing the syringe for disposal.
Embodiment 46. A method with the features of embodiment 44 wherein step (b) comprises robotically grasping the cylinder of the selected syringe.
Embodiment 47. A method with the features of embodiment 44 further comprising repeating steps (b-d) with a different syringe removed from the rack.
Embodiment 48. A method of mixing materials, said method comprising the steps of
   mixing material in a container having a bottom wall and an open top by rotating at least one mixing blade positioned inside the container above the bottom wall,
   causing the at least one mixing blade to stop at a predetermined rotary position to provide a predetermined unobstructed path extending from the open top of the container to the bottom wall of the container,
   robotically moving an aspirator down along said predetermined unobstructed path to an aspirating position adjacent the bottom wall of the container, taking into account the predetermined rotary position of the at least one mixing blade so that there is no contact between the blade and the aspirator as the aspirator moves to said aspirating position, and
   aspirating material from the container.
Embodiment 49. A method with the features of embodiment 48 wherein said mixing step comprises rotating the at least one mixing blade by a direct drive mechanism extending up through the bottom wall of the container.
Embodiment 50. A method with the features of embodiment 49 further comprising performing said mixing step simultaneously in a plurality of containers, and aspirating material from at least one of said containers.
Embodiment 51. A method with the features of embodiment 50 further comprising robotically dispensing material into at least one container of said plurality of containers, and then robotically moving the container to a weigh station and weighing the container and materials therein.
Embodiment 52. A robotic work station for mixing materials, comprising
   a robot,
   an aspirator carried by the robot,
   a container at a mixing station for holding materials to be mixed, said container having a bottom wall and an open top,
   a mixer for mixing the contents of the container, said mixer comprising at least one mixing blade positioned inside the container above the bottom wall of the container,
   a drive system for rotating the at least one mixing blade, and
   a controller for controlling the drive system to stop the at least one mixing blade in the container at a predetermined rotary position to provide an unobstructed path extending from the open top of the container to the bottom wall of the container, and for controlling the robot to move the aspirator down along said unobstructed path to an aspirating position adjacent the bottom wall of the container, taking into account the predetermined rotary position of the at least one mixing blade so that there is no contact between the blade and the aspirator as the aspirator moves to said aspirating position.
Embodiment 53. A robotic work station with the features of embodiment 52 further comprising a plurality of said containers at the work station, said controller being programmed for operating the robot to aspirate material from each of the containers.
Embodiment 54. A robotic work station with the features of embodiment 53 wherein said drive system comprises a plurality of direct drive mechanisms extending up through the bottom walls of the containers.
Embodiment 55. A robotic work station with the features of embodiment 54 wherein each direct drive mechanism comprises a mixing shaft extending up through the bottom wall of a respective container, a drive shaft for rotating the mixing shaft, and a releasable coupling for releasably connecting the drive shaft to the mixing shaft.
Embodiment 56. A robotic work station with the features of embodiment 55 wherein said drive system further comprises a common motor for rotating said drive shafts.
Embodiment 57. A robotic work station with the features of embodiment 56 further comprising a weighing device at a weigh station for weighing the container and material therein, said robot being programmed to move the container between said mixing and weigh stations.

## Claims

1. Apparatus for actuating one or more syringes, each syringe comprising a cylinder having upper and lower ends, and a plunger movable in the cylinder, said apparatus comprising
a programmable robot, and
at least one syringe actuating device mounted on the robot for actuating a syringe to transfer material to or from a container;
said at least one syringe actuating device comprising
a holder for holding and releasing the cylinder of a syringe,
an actuator movable relative to the holder, and
a coupling for coupling the actuator to the plunger of the syringe such that, when the cylinder of the syringe is held by the holder, movement of the actuator in a first direction causes the plunger to move up in the cylinder and movement of the actuator in a second direction causes the plunger to move down in the cylinder.

2. Apparatus according to claim 1 wherein said robot comprises an arm movable in a generally horizontal plane along X and Y axes, and a Z-member mounted on the arm for generally vertical movement along a Z axis, said syringe actuating device being mounted on the Z-member of the robot.

3. Apparatus according to claim 2 wherein said at least one syringe actuating device is movable relative to said Z-member in a generally Z-direction.

4. Apparatus according to claim 3 wherein said at least one syringe actuating device is mounted on a carriage movable on a rail secured in fixed position relative to said Z-member.

5. Apparatus according to claim 2 further comprising a sensing device mounted on the Z-member of the robot adjacent said at least one syringe actuating device and moveable relative to the Z-member in a generally Z-direction.

6. Apparatus according to claim 1 further comprising a sensing device mounted on the robot adjacent said at least one syringe actuating device, said sensing device being operable to sense a property of material in said container.

7. Apparatus according to claims 1 or 6 comprising at least two of said syringe actuating devices mounted adjacent one another on the robot.

8. Apparatus according to claim 1 wherein said holder comprises a housing having an open lower end to permit a syringe to be entered into the housing to a position to be held by the holder and to exit the housing upon release by the holder, and at least one holding member mounted on the housing for movement between holding and release positions.

9. Apparatus according to claim 8 wherein said at least one holding member is configured for engagement with a flange on the cylinder of the syringe when the holding member is in said holding position.

10. Apparatus according to claim 8 further comprising a release mechanism on the housing adapted to be actuated for moving the at least one holding member to said release position.

11. Apparatus according to claim 10 further comprising a deck, a rack on the deck for supporting a plurality of syringes in position for loading into the housing of the holder, and a syringe release device on the deck configured to interact with said release mechanism to release a syringe from the housing for disposal.

12. Apparatus according to claim 1 or 8 wherein said coupling is a releasable coupling movable between a coupling position for coupling the actuator to the plunger of the syringe and a de-coupling position for de-coupling the actuator from the syringe.

13. Apparatus according to claim 12 wherein the coupling is movable to its de-coupling position in response to movement of the at least one holding member toward said release position.

14. Apparatus according to claim 13 further comprising linkage operable in response to movement of the at least one holder member toward said release position to move the coupling to its de-coupling position.

15. A method for actuating one or more syringes, using the apperatus according to any of claims 1 through 14.
